# EUROPEAN PATENT SPECIFICATION

(11) **EP 2 695 945 B1**
(45) Date of publication and mention of the grant of the patent: **22.07.2015**
(21) Application number: 13191475.6
(22) Date of filing: 28.04.2010
(51) Int. Cl.: C12N 15/861

(54) **Methods and reagents for efficient and targeted gene transfer to cells of monocyte-macrophage lineage**
Verfahren und Reagenzien zum effizienten und gezielten Gentransfer in Zellen der Monozyten-Makrophagen Zelllinie
Procédés et réactifs pour le transfert génétique efficace et ciblé vers des cellules de lignage monocyte-macrophage

(30) Priority: 29.04.2009 EP 09159001
(43) Date of publication of application: 12.02.2014
(62) Divisional of application: 10719310.4
(73) Proprietor: Universitat Autònoma De Barcelona, 08193 Bellaterra, Barcelona (ES); Grifols, S.A., 08022 Barcelona (ES); Fundació Institut d'Investigació en Ciències de la Salut Germans Trias i Pujol, 08916 Badalona, Barcelona (ES); Fundació Privada Institució Catalana de Recerca I Estudis Avançats, 08010 Barcelona (ES)
(72) Inventor: Gassull Duro, Miquel Angel, 08916 Badalona (Barcelona) (ES); Rio Fernandez, Adolfo, 08174 Sant Cugat del Valles (Barcelona) (ES); Fernandez Gimeno, Ester, 08193 Bellaterra (Barcelona) (ES); Chillon Rodriguez, Miguel, 08010 Barcelona (ES)
(74) Representative: Durán-Corretjer, S.L.P.

(56) References cited:
- WO-A-2008/026015
- WO-A2-02/24730
- LEMIALE F ET AL: "Novel adenovirus vaccine vectors based on the enteric-tropic serotype 41", VACCINE, ELSEVIER LTD, GB, vol. 25, no. 11, 13 February 2007 (2007-02-13), pages 2074-2084, XP005886423, ISSN: 0264-410X, DOI: 10.1016/J.VACCINE.2006.11.025
- OKADA N ET AL: "Efficient antigen gene transduction using Arg-Gly-Asp fiber-mutant adenovirus vectors can potentiate antitumor vaccine efficacy and maturation of murine dendritic cells", CANCER RESEARCH, AMERICAN ASSOCIATION FOR CANCER RESEARCH, US, vol. 61, no. 21, 1 November 2001 (2001-11-01), pages 7913-7919, XP002994946, ISSN: 0008-5472
- SCHOGGINS JOHN W ET AL: "Subgroup B and F fiber chimeras eliminate normal adenovirus type 5 vector transduction in vitro and in vivo", JOURNAL OF VIROLOGY, THE AMERICAN SOCIETY FOR MICROBIOLOGY, US, vol. 77, no. 2, 1 January 2003 (2003-01-01), pages 1039-1048, XP002581919, ISSN: 0022-538X, DOI: 10.1128/JVI.77.2.1039-1048.2003
- NICOL C G ET AL: "Effect of adenovirus serotype 5 fiber and penton modifications on in vivo tropism in rats", MOLECULAR THERAPY, ACADEMIC PRESS, SAN DIEGO, CA, US, vol. 10, no. 2, 1 August 2004 (2004-08-01) , pages 344-354, XP004660571, ISSN: 1525-0016
- NICKLIN ET AL: "The influence of adenovirus fiber structure and function on vector development for gene therapy", MOLECULAR THERAPY, ACADEMIC PRESS, SAN DIEGO, CA, US, vol. 12, no. 3, 1 September 2005 (2005-09-01), pages 384-393, XP005472372, ISSN: 1525-0016
- KASHENTSEVA E A ET AL: "Targeting of Adenovirus Serotype 5 Pseudotyped with Short Fiber from Serotype 41 to c-erbB2-Positive Cells using Bispecific Single-Chain Diabody", JOURNAL OF MOLECULAR BIOLOGY, LONDON, GB, vol. 388, no. 3, 13 March 2009 (2009-03-13), pages 443-461, XP026046493, ISSN: 0022-2836 [retrieved on 2009-03-13]
- SHAYAKHMETOV DMITRY M ET AL: "A high-capacity, capsid-modified hybrid adenovirus/adeno-associated virus vector for stable transduction of human hematopoietic cells", JOURNAL OF VIROLOGY, vol. 76, no. 3, February 2002 (2002-02), pages 1135-1143, XP002542774, ISSN: 0022-538X

## Description

### FIELD OF INVENTION

The present invention relates to the ability of chimeric human adenovirus 5 carrying the short fiber of enteric Ad40 to transfer genetic material to monocytes and macrophages in an efficient and selective process, and the optimization of the dose-response and the biosafety profile in transduced cells.

### BACKGROUND OF INVENTION

Mononuclear cells have been defined as a hematopoietic cell lineage derived form progenitor cells in the bone marrow. Committed myeloid progenitor cells differentiate to form blood monocytes, which circulate in the blood and then enter the tissues to become resident macrophages. The existence of monocyte subsets in humans has been known and studied for many years. Human monocytes were identified by the expression of CD14. They can be further classified on the basis of CD16 expression (the high affinity Fc receptor). CD16- cells are referred to as classical monocytes since they are ordinarily about 90 % of total monocytes in healthy individuals. CD16+ cells appear to be expanded in many inflammatory diseases and exhibit a preferential migration across the endothelial layers in response to chemokines. They are thus usually referred to as non-classical or proinflammatory monocytes. This monocyte subset has been shown to be able to differentiate into dendritic cells (DC). A considerable increase in the number of CD14+CD16+ monocytes has been described for a variety of systemic and localized infections.

Whereas monocytes are widely recruitable cells, most of the migratory ability of monocyte derived macrophages and DC abrogates in the maturation process due to a progressive loss of inflammatory chemokine receptors. Conversely, the up regulation of CCR7 during DC maturation increases its migratory response towards lymphatic vessels and draining lymph nodes. (Eur. J. Immunol 1998. 28:2760-2769; J. Neuroimmunol 2008 197:21-28).

Thus, the genetic manipulation of blood monocytes is highly attractive since it may allow the manipulation of the immune response in a particular location in which an inflammatory process takes place and to which monocytes will preferably migrate under the influence of chemotactic factors released by damaged cells in inflamed sites.

Monocytes occur in human blood in varying amounts, ranging from 5-10% of total peripheral blood leukocytes in healthy individuals, but this number can be greatly exceeded whenever an inflammatory condition is present. They vary in size and have different degrees of granularity and varied nuclear morphology. Indeed, monocytes are recruited preferably to inflamed areas, where they gain new roles and accomplish phagocytic and antigen presenting functions. Under certain conditions they may evolve to dendritic cells, which are highly efficient antigen presenting cells and which orchestrate the immune response elicited in front of a particular insult and drive lymphocyte function.

Macrophages are relatively resistant to transfection but there are several reports claiming that adenoviral vectors may eventually be used as vectors to transfect monocyte-derived macrophages, dendritic cells or even monocytes (Eur. J. Immunol. 1999, 29: 964-972; Blood 1998 91: 392-398; Acta Pharmacol. Sin. 2006, 27: 609-616; Biochem. Biophys. Res. Com. 1993, 195: 1174-1183; Gene Ther. 2000, 7: 263-270, Cytotherapy, 2006 8: 36-46; Mol. Ther. 2007, 16: 210-217 and J. Immunol. 2008, 181: 8018-8026). However, these reports do not use undifferentiated and unstimulated monocytes since they culture cells in presence of different combinations of cytokines and factors such as Macrophage colony stimulating factor (M-CSF), also know as Colony stimulating factor-1 (CSF-1), Granulocyte-macrophage colony stimulating factor (GM-CSF) and others. In contact with monocytes, M-CSF enhances the expression of differentiation antigens, increases chemotactic and phagocytic activities and stimulates the production of several cytokines (Ross J. A. and Auger M. J. in The Macrophage, Second Edition; Oxford University Press, 2002; Pathol. Int. 2008, 58: 143-155). Furthermore, it has been demonstrated that human DC can be generated from monocytes in the presence of GM-CSF alone (Immunobiology 2008, 213: 859-870). In contrast, monocytes are essentially considered as non-transfectable cells (Gene Ther. 1997, 4: 524-532; J. Immunol. Methods 2008, 330: 86-95). So, to date the attempts to selectively transfect peripheral blood undifferentiated and unstimulated monocytes using either electroporation or non-viral and viral methods have been unsuccessful (Gastroenterology 2006 131:1799-181). Of note, recent use of chimeric Ad5 vectors carrying fibers of Ad35 and Ad11 has allowed infection of different primary blood cells including T-cells, B-cells and monocytes (Virology 2006, 25: 349(1): 96-111). Moreover, Herold et al., (Langenback's Archives of Surgery, 2006, 391:72-82) have also reported the use of adenovirus for transfecting monocytes, Gerstzen et al., (J. Biol. Chem. 2001, 276: 26846-26851) and Kaan-Shanzer et al., 2001, Hum. Gene Therapy, 12:1989-2005) have reported that adenoviruses can be used for transducing umbilical cord blood moncytes. Mayne et al (J. Immunol. Methods, 2003, 278: 45-56) have described that the efficiency of the transfection of CD14+ cells with adenoviruses can be improved if the cells are centrifuged in the presence of adenoviruses prior to the transducing step.

The international patent application WO9850053 describes methods for transducing monocytes using chimeric adenoviral vectors using recombinant Ad5 adenovirus comprising the Ad3 fiber protein. This methods allows a transduction efficiency of 80% (at 100 infective particles/cell) or 100% (at 1000 100 infective particles/cell).

However, none of these vectors are particularly efficient for use in vivo since they are capable of transducing other cell types thus resulting in secondary effects due to the infection of non-target cells. Thus, there is a need in the art of methods and reagents which allow the selective and efficient transduction of uncommitted, unstimulated and undifferentiated peripheral blood monocytes.

### SUMMARY OF THE INVENTION

The invention relates to in vitro methods and nanotransporters for medical uses as defined in the claims.

Furthermore described herein is a nanotransporter comprising at least a part of the short fiber protein of a subgroup F adenovirus or a functionally equivalent variant thereof with the proviso that the nanotransporter is not an adenoviral particle.

Furthermore described is an *in vitro* method for delivering a compound of interest to a cell of the monocyte-macrophage lineage which comprises contacting said cell with a nanotransporter carrying said compound of interest and wherein the transporter contains at least a part of the short fiber protein of a group F adenovirus or a functionally equivalent variant thereof.

Furthermore described is a nanotransporter comprising a product of interest and at least a part of the short fiber protein of a subgroup F adenovirus or a functionally equivalent variant thereof for use in medicine.

Furthermore described is a nanotransporter comprising a product of interest and at least a part of the short fiber protein of a subgroup F adenovirus or a functionally equivalent variant thereof for use in the treatment of a disease associated with cells of the monocyte-macrophage lineage.

### BRIEF DESCRIPTION OF THE FIGURES

Figure 1 shows the percentage of infection of human adenovirus 5 vectors and chimeric adenovirus 5/40 in the murine monocyte cell line RAW 264.7.
Figure 2 shows the percentage of infection of human adenovirus 5 vectors and chimeric adenovirus 5/40 in the human monocyte cell lines THP-1 (high infectivity) and U-937 (low infectivity).
Figure 3 shows the proportions of the different blood cell populations in human controls. T lymphocytes are the main population (representing 87% of the total). Monocytes represent only 8% of total mononuclear cells in the blood.
Figure 4 shows the percentage of infection of human adenovirus 5 vectors and chimeric adenovirus 5/40 in the different blood cell populations. The cell populations were identified by membrane specific markers using fluorescence analysis (FACS): CD3+ (T lymphocytes), CD19 + (B lymphocytes), CD56+ / CD16+ / CD3- (NK) cells, CD14+ (monocytes).
Figure 5 shows how proinflammatory monocytes (CD14+/CD16+) and classical monocytes (CD14+ / CD16-) are equally infected by adenoviral vectors.
Figure 6 shows how the chimeric adenovirus 5/40 allows a GFP higher transgene expression than that from the adenovirus 5, either in (CD14+/CD16-) or in (CD14+/CD16+) monocytes.
Figure 7 shows how the chimeric adenovirus 5/40 allows a greater GFP transgene expression respect to the adenovirus 5, either in (CD14+/CD16-) or in (CD14+/CD16+) monocytes. Ad5: Continuous line. Ad5/40: Discontinuous line.
Figure 8 shows the percentage of infection of monocytes (CD14+) at different doses of viral particles per cell. Ad5/40: Continuous line. Ad5: Discontinuous line.
Figure 9 shows the viability of monocytes (CD14+) depending on the dose of adenovirus Ad5 and Ad5/40 used. Ad5/40: Continuous line. Ad5: Discontinuous line.

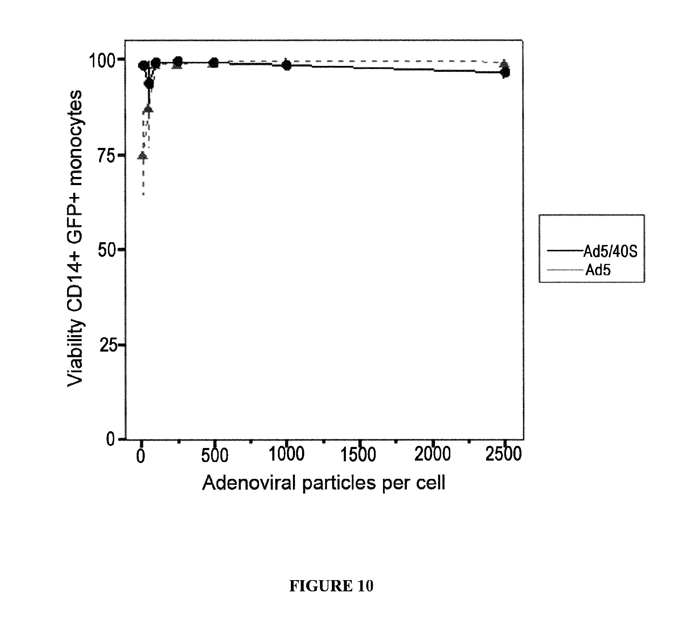

Figure 10 shows the viability of monocytes (CD14+) expressing the GFP transgene depending on the dose of adenovirus Ad5 and Ad5/40 used. Ad5/40: Continuous line. Ad5: Discontinuous line.

### DETAILED DESCRIPTION OF THE INVENTION

The authors of the present invention have observed that, surprisingly, undiferenciated and unstimulated monocytes, a cell line hitherto thought to be refractory to transfection, could be indeed transfected using chimeric human adenovirus vectors 5 carrying the short fiber of enteric Ad40. In fact, as shown under examples 1 to 3 of the present invention, a recombinant Ad5 adenovirus comprising the short fibre protein of Ad40 is capable of transfecting mouse monocytes of the intestinal mucosa, monocyte-derived human macrophages and undiferenciated and unstimulated peripheral blood monocytes. The ability of the Ad5/40 chimeric adenovirus to transfer genetic materials to monocytes seems to rely on the presence within the virus of the Ad40 short fiber, as shown by the fact that Ad5 carrying its own fiber protein are much less efficient in transfecting monocytes (see example 5 of the invention). This finding opens the door for developing fiber protein of a subgroup F adenovirus.

### A. NANOTRANSPORTERS OF THE INVENTION

Described herein is a nanotransporter comprising at least a part of the short fiber protein of a subgroup F adenovirus or a functionally equivalent variant thereof.

The term "nanotransporter", as used herein, relates to a multi-component complex with controlled dimensions, e.g., a diameter or radius on the order of about 1 to about 1000 nanometers that contains a compound of interest. Preferred nanotransporters for use in the present invention include viruses, virus-like particles (VLP), nanoparticles, protein cages and the like. In the case that the nanotransporter is a virus, then the virus is preferably a virus different from a subgroup F adenovirus.

### a. Viral nanotransporters

In one embodiment, the nanotransporter of the invention is a virus. The skilled person will appreciate that any virus known in the art may be used as nanotransporter in the present invention provided that sufficient information is available so as to allow the modification of the external components by the short fiber protein of a subgroup adenovirus. Thus, in the case of non-enveloped viruses, the nanotransporters of the invention are obtained by directly modifying at least one the capsid proteins, either by chemical coupling of the short fiber protein or by inserting the sequence encoding the short fiber protein into the viral gene coding for the capsid protein so that, upon synthesis and assembly into the capsid, the short fiber protein is exposed to the outer surface of the capsid. Examples of suitable virus capsids that can be modified in the above manner include, but are not limited to, capsids from sindbis and other alphaviruses, rhabdoviruses (e.g. vesicular stomatitis virus), picornaviruses (e.g., human rhino virus, Aichi virus), togaviruses (e.g., rubella virus), orthomyxoviruses (e.g., Thogoto virus, Batken virus, fowl plague virus), polyomaviruses (e.g., polyomavirus BK, polyomavirus JC, avian polyomavirus BFDV), parvoviruses, rotaviruses, bacteriophage Qβ, bacteriophage P1, bacteriophage M13, bacteriophage MS2, bacteriophage G4, bacteriophage P2, bacteriophage P4, bacteriophage 186, bacteriophage Φ6, bacteriophage Φ29, bacteriophage MS2, bacteriophage N4, bacteriophage ΦX174, bacteriophage AP205, Norwalk virus, foot and mouth disease virus, a retrovirus, Hepatitis B virus, Tobacco mosaic virus (TMV), satellite panicum mosaic virus (SPMV), flock house virus and human papilomavirus.

Alternatively, wherein the nanotransporter is an enveloped virus, the short fiber protein is preferably attached to or replacing a part of the envelope glycoproteins. Some non-limiting examples of surface glycoproteins that may be used for inserting the short fiber protein include glycoproteins from alphaviruses, such as Semliki Forest virus (SFV), Ross River virus (RRV) and Aura virus (AV), which comprise surface glycoproteins such as E1, E2, and E3. The E2 glycoproteins derived from the Sindbis virus (SIN) and the hemagglutinin (HA) of influenza virus are non-retroviral glycoproteins that specifically bind particular molecules on cell surfaces (heparin sulfate glycosaminoglycan for E2, sialic acid for HA) which are known to tolerate certain genetic modifications and remain efficiently assembled on the retroviral surface (Morizono et al. J. Virol. 75, 8016-8020); glycoproteins of Lassa fever virus, Hepatitis B virus, Rabies virus, Borna disease virus, Hantaan virus, or SARS- CoV; flavivirus-based surface glycoproteins, hemagglutinin (HA) from influenza A/fowl plague virus/Rostock/34 (FPV), a class I fusogen, is used (T. Hatziioannou, S. Valsesia-Wittmann, S. J. Russell, F. L. Cosset, J. Virol. 72, 5313 (1998)). Suitables viruses for use in the present invention comprise alphaviruses, paramyxoviruses, rhabdoviruses, coronaviruses, picornaviruses, myxoviruses, reoviruses, bunyaviruses, flaviviruses, rubiviruses, filoviruses, arenaviruses, arteriviruses or caliciviruses.

Suitable viruses that can be modified by the insertion of the subgroup F adenovirus short fiber protein include retrovirus, adenovirus, parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g. measles and Sendai), positive strand RNA viruses such as picornavirus and alphavirus, and double stranded DNA viruses including adenovirus, herpesvirus (e.g., Herpes Simplex virus types 1 and 2, Epstein-Barr virus, cytomegalovirus), and poxvirus (e.g., vaccinia, fowlpox and canarypox). Other viruses include, for example, Norwalk viruses, togaviruses, flaviviruses, reoviruses, papovaviruses, hepadnaviruses, and hepatitis viruses. Examples of retroviruses include avian leukosis-sarcoma viruses (e.g., avian leukosis viruses, avian sarcoma viruses), mammalian C-type, B-type, D-type retroviruses, HTLV-BLV viruses, lentiviruses, spumaviruses (Coffin, J. M., "Retroviridae: The viruses and their replication", in Fundamental Virology, Third Edition, edited by B. N. Fields, D. M. Knipe, P. M. Howley, et al. Lippincott-Raven Philadelphia, Pa. (1996)). Other examples include murine leukemia viruses, murine sarcoma viruses, mouse mammary tumor viruses, bovine leukemia viruses, feline leukemia viruses, feline sarcoma viruses, avian leukemia viruses, human T-cell leukemia viruses, baboon endogenous viruses, Gibbon ape leukemia viruses, Mason Pfizer monkey viruses, simian immunodeficiency viruses, simian sarcoma viruses, Rous sarcoma viruses and lentiviruses.

Retroviruses contain two envelope glycoprotein subunits (designated surface or SU and transmembrane or TM) which form an oligomeric complex on the viral surface and mediate viral entry. The SU protein contains the viral receptor binding determinants whereas the TM protein contains a hydrophobic transmembrane region and a separate hydrophobic segment that mediates virus-cell membrane fusion (Weiss, R. A., "Cellular receptors and viral glycoproteins involved in retrovirus entry," pp. 1-107, in J. A. Levy (ed.), The Retroviridae, Vol. 2., Plenum Press: New York, N.Y. (1993)). The first step of infection is the binding of the viral particle via the surface protein of the retrovirus envelope (env) protein and viral and cellular membrane fusion for viral uptake via the transmembrane protein of the env protein. The env protein is largely responsible for the specificity (between cell-types and between species) of the infectivity of retroviral vectors. If retroviruses are used as nanotransporters according to the present invention, the subgroup F adenoviral short fiber protein can be inserted into any of the envelope glycoprotein subunit or into the env glycoprotein.

Adeno-associated viruses (AAV) have a linear single-stranded DNA genome and their receptor has not yet been described. These viruses only undergo productive infection if the infected cells are co-infected with a helper virus (e.g., adeno- or herpesvirus) otherwise the genome becomes integrated in a latent state at a specific site on a human chromosome (Linden, Proc. Natl. Acad. Sci. USA, 93:11288-11294 (1996); and Bems, K. J., "Parvoviridae: The viruses and their replication" in Fields Virology, Third Edition, edited by B. N. Fields, D. M. Knipe, P. M. Howley et al., Lippincott-Raven Publishers: Philadelphia, Pa. (1996)). Recombinant adeno-associated viruses are typically made by replacing viral genes with desired genes of interest or by simply adding the terminal AAV DNA sequences (ITRs) to these genes.

Alternatively, the nanotransporters of the present invention may be based on negative strand RNA viruses. These viruses are capable of infecting cells by a variety of different mechanisms. For example, Influenza A viruses which have a segmented RNA genome, contain a surface hemagglutinin protein which binds to cell surface sialic acid receptors and mediates viral entry in a low pH endosome following receptor-mediated endocytosis. The invention contemplates the modification of the surface HA by the insertion of the adenoviral short fiber protein. Paramyxoviruses which have a non-segmented RNA genome have two surface viral proteins, the hemagglutinin (HN) and fusion protein (F), required for viral entry which occurs at neutral pH. These viruses can utilize sialic acid receptors, or protein receptors (e.g., CD46 used by measles virus), for viral entry. The adenoviral short fiber protein may be inserted or attached to either hemagglutinin (HN) or to the fusion protein (F).

Rhabdoviruses (e.g., VSV) which have a non-segmented RNA genome, contain a surface protein (G) which binds to specific cell surface receptors and mediates viral entry in a low pH endosome. A specific phospholipid appears to be one of the receptors for VSV. The adenoviral short fiber protein may be inserted or attached to the G protein.

In another embodiment, the nanotransporters of the invention may be based on positive strand RNA viruses. These viruses infect cells by different mechanisms. For example, among the picornaviruses, different members of the immunoglobulin protein superfamily are used as cellular receptors by poliovirus, by the major subgroups of rhinoviruses, and by coxsackie B viruses, whereas an integrin protein is used by some types of ecoviruses and a low density lipoprotein receptor is used by minor subgroups of rhinoviruses. Following receptor-binding, it is not yet known precisely what role receptor-mediated endocytosis plays for picornaviral entry, if indeed it is required. Because the picornaviruses lack a surface lipid bilayer, their entry pathway does not involve fusion of a viral membrane with a host cell membrane. Thus, if picornaviruses are to be used as nanotransporters, the adenoviral short fiber protein should be attached or coupled to a capsid protein such as the viral proteins VP1, VP2, VP3 and VP4.

In contrast, the alphaviruses (e.g., Sindbis virus and Semliki virus) do contain a surface lipid bilayer. These viruses contain two (E1 and E2) surface proteins, and in some cases a third (E3) surface protein important for viral entry. These viruses use various cell surface receptors. For example, Sindbis virus can use a laminin receptor or other receptors and generally enter cells by a pH-dependent mechanism, following receptor-mediated endocytosis. Thus, nanotransporters according to the invention based on alphavirus vectors may contain the adenoviral short fiber protein attached or inserted within the E1 and/or E2 surface proteins.

Another possibility is the use of herpesviruses as nanotransporters according to the present invention. Herpesviruses have large double-stranded DNA genomes which contain a number of surface glycoproteins involved in viral entry and utilize various cell surface receptors. For example, herpes simplex virus and cytomegalovirus entry involves binding to a heparin sulfate cell surface receptor and herpes simplex viruses use other proteins (e.g., HVEM) for viral entry. In contrast, Epstein-Barr virus entry is initiated by binding to a completely distinct cell surface receptor, CR2 (Wolf, Intervirology 35:26-39 (1993)). Strategies have been described that allow one to engineer herpes simplex viruses, cytomegaloviruses and Epstein-Barr viruses as vectors for heterologous gene expression.

In another embodiment, the invention contemplates the use of poxviruses as nanotransporters. Poxviruses have large double stranded DNA genomes and enter cells by a pH-independent mechanism via receptors that remain to be defined. Poxvirus vectors have been used extensively for the expression of heterologous recombinant genes and as vaccines (Moss, B., Proc. Natl. Acad. Sci. USA, 93:11341-11348 (1996); and Paoletti, Proc. Natl. Acad. Sci. USA, 93:11349-11353 (1996)).

In another embodiment, the nanotransporters are pseudotyped retroviruses wherein the env protein from the retrovirus is replaced by the adenoviral short fiber protein. Typically, retroviral vectors are manufactured by "packaged cell lines" which provide the retroviral proteins necessary for infection (e.g., env, gag and pol), but are incapable of replication upon infection. See, for example, Miller, AD, Current Topics in Microbiology and Immunology, Vol. 158, pp. 1-24 (1992). Suitable retroviral vectors for use with the invention include, without limitation, Moloney murine leukemia virus (MLV)-derived vectors, and include more complex retrovirus-derived vectors, e.g., lentivirus-derived vectors. Human Immunodeficiency virus (HIV-1)-derived vectors belong to this category. Other examples include lentivirus vectors derived from HIV-2, feline immunodeficiency virus (FIV), equine infectious anemia virus, simian immunodeficiency virus (SIV) and maedi/visna virus.

In a more preferred embodiment, the nanotransporters of the invention are adenoviruses. In this case, the adenoviruses are not subgroup F adenoviruses of the same type as the short fiber protein, i.e. if the short fiber protein derived from an Ad40 adenovirus, the nanotransporter may be other adenoviruses with the exception of Ad40. Conversely, if the short fiber protein derived from an Ad41 adenovirus, the nanotransporter may be any adenovirus with the exception of Ad41. Adenoviruses have a linear double-stranded DNA genome. Adenoviruses infection of target cells occurs by three physically distinct receptor-ligand interactions. First the knob domain of the viral surface fiber protein binds specifically to a cell surface receptor. In the case of human HeLa cells, the receptor for adenoviruses 2 and 5 is designated CAR, a member of the immunoglobulin protein superfamily, which also serves as a cellular receptor for coxsackie B viruses (Bergelson, 1996, Science, 275:1320-1323). Second, interaction takes place between the the viral penton base protein and alphaV integrins. A third receptor-binding site is localized to the third beta-spiral repeat in the fiber shaft and mediates binding to heparan sulphate glycosaminoglycans (HSG). Other polypeptides present on the surface of adenoviral particles include the capsomer proteins which include the major component hexons as well as minor amounts of pIIIa and pIX.

Alteration of adenoviral targeting has been carried out by modification of capsomer proteins (Campos and Barry, 2006, Virology, 349:453-462) albeit with lower efficiency than by modification of the fiber protein. Thus, while the present invention foresees the attachment or fusion of the short fiber protein any adenoviral surface protein, it is preferred that the attachment or fusion is done onto at least one of the surface polypeptides responsible for binding to the target cell. Thus, preferably, the adenovirus may be modified either by inserting or attaching the subgroup F adenoviral fiber protein to the penton base or, more preferably, to the adenoviral fiber protein itself.

Adenovirus fibers are trimeric proteins that consist of a globular C-terminal domain (the "knob" domain), a central fibrous shaft and an N-terminal part (the "tail" domain) that attaches to the viral capsid. In the presence of the globular C-terminal domain, which is necessary for correct trimerization, the shaft segment adopts a triple beta-spiral conformation. Fiber proteins are incorporated as trimers into the capsid structure. The skilled person will appreciate that the incorporation of the short fiber protein into the adenoviral protein can be carried out in different manners, depending on whether the native tropism of the adenovirus is to be preserved or is to be abolished.

In a preferred embodiment, it is possible to add the short fiber proteins to the C- terminus of fiber as described (Michael et al., 1995, Gene Therapy, 2:660-668 and Wickham, T.J., et al., 1997, J. Virol., 71: 8221-8229). Alternatively, it is possible to incorporate inserts into the HI-loop of the fiber knob as previously described (Nicklin et al., 1998, J. Virol., 72: 1844-1852 and Krasnykh, V., et al., 1998, J. Virol., 72: 1844-1852), an approach that has been shown to tolerate introduction of certain peptides larger than 100 residues without substantially affecting propagation and infectivity of the resulting AdVs. Moreover, it is also possible to insert the short fiber protein within a recombinant spike molecule in which the fiber knob domain alone or in combination with (part of) the fiber shaft domain has been replaced with an exogenous trimerization domain as described by van Beusechem, V. W., et al., (Gene Ther., 2000, 7: 1940-1946 and Magnusson et al. (J. Virol., 2001, 75: 7280-7289). This approach has the advantage that it allows to broaden the range of possible targeting epitopes as well as removes native binding sites residing in the fiber knob. Recombinant spike molecules are referred to herein as "knobless fibers" or "chimeric adenovirus spike proteins". A knobless fiber molecule or chimeric adenovirus spike protein is defined in that it essentially lacks a functional fiber knob domain, is capable of forming trimers and is capable of attaching onto an adenovirus capsid. A "knobless fiber" does thus not mean that the molecule is a fiber protein lacking the knob domain. While this may be the case, other regions of the fiber, such as the shaft domain or part thereof, may also be lacking. A chimeric adenovirus spike protein of the invention may further comprise additional sequences such as targeting sequences and/or spacer/linker sequences. The "trimerization" domain of the fiber protein is, as mentioned, located in the knob domain. If the knob domain is removed from the fiber thereby creating a knobless fiber, it is preferred that the lost trimerization function is replaced by other sequences comprising a so-called "trimerization domain". Otherwise, no trimers are formed and no fiber incorporated into the adenovirus particle. In the art different trimerization domains have been produced to replace the adenovirus trimerization domain. Heterologous trimerization domains can be derived from many different kinds of proteins. Non-limiting examples of knobless fiber proteins of the invention are described in WO01/81607, in WO01/02431 and in WO 98/54346.

The fiber "tail" domain provides the attachment function of the fiber to the adenovirus capsid. This attachment function is provided by a nuclear localization sequence, to transport the fiber to the nucleus where the adenovirus particles are assembled, and a recognition sequence for binding the fiber to penton base proteins in the adenovirus capsid. It is preferred that a knobless fiber of the invention comprises at least a functional part of this tail domain, where functional means providing capacity to bind to the adenovirus capsid when expressed in a cell. A knobless fiber of the present invention thus preferably comprises an adenovirus fiber "tail" domain and a heterologous and/or non-adenovirus trimerization domain. For means and method for producing knobless fiber containing adenoviruses reference is made to WO01/81607.

Recombinant adenoviral vectors are generated by a variety of techniques that include introducing a desired gene of interest into a bacterial plasmid at a site flanked by adenovirus sequences. These sequences provide control elements for gene expression and serve as sites for recombination with a compatible adenoviral genome when cotransfected together into an appropriate mammalian cell line (Horwitz, M. S., "Adenoviruses," in Fields Virology, Third Edition, edited by B. N. Fields, D. M. Knipe, P. M. Howley et al., Lippincott-Raven Publishers: Philadelphia, Pa. (1996)).

Thus, the invention contemplates the use of any known adenoviral serotype known in the art and including, without limitation, any of the serotypes as defined in table 1.

**Table 1: Some exemplary adenoviral subgroups and serotypes suitable for use as nanotransporters in the present invention.**

| Subgroup | Serotypes |
|---|---|
| A | 12, 18, 31 |
| B | 3, 7, 11, 14, 16, 21, 34, 35 |
| C | 1, 2, 5, 6 |
| D | 8, 9, 10, 13, 15, 17, 19, 20, 22-30, 32, 33, 36-39, 42-47 |
| E | 4 |
| F | 40, 41 |

In a preferred embodiment, the adenovirus belongs to serotype 5 (Ad5) and has been modified by incorporating the adenovirus 40 short fiber protein (hereinafter referred to as Ad5/40).

### b. Nanotransporters based on VLPs

In one embodiment, the nanotransporter used in the present invention is a VLP. The term "VLP or viral-like particle", as used herein, relates to a self-assembling macromolecular structure formed by the viral nucleocapsids which acquire a quaternary structure resembling that of the virus from which they originate but which are devoid of the virus genetic material.

The VLPs for use according to the present invention may be formed from polypeptides derived from any virus known in the art having an ordered and repetitive structure. VLPs can be produced and purified from virus-infected cell culture. The resulting virus or virus-like particle for vaccine purpose needs to be devoid of virulence. Besides genetic engineering, physical or chemical methods can be employed to inactivate the viral genome function, such as UV irradiation, formaldehyde treatment. Alternatively, the VLP is a recombinant VLP. The skilled person will appreciate that almost all commonly known viruses of known sequence may be used for the generation of VLPs provided that the gene encoding the coat protein or proteins can be easily identified by a skilled artisan. The preparation of VLPs by the recombinant expression of the coat protein or proteins in a host is within the common knowledge of a skilled artisan. Suitable VLPS can be obtained from the nucleocapsid proteins of a virus selected form the group consisting of RNA-bacteriophages, adenovirus, papaya mosaic virus, influenza virus, norovirus, papillomavirus, hepadnaviridae, respiratory syncytial virus, hepatitis B virus, hepatitis C virus, measles virus; Sindbis virus; rotavirus, foot-and-mouth-disease virus, Newcastle disease virus, Norwalk virus, alphavirus; SARS, paramoxyvirus, transmissible gastroenteritis virus retrovirus, retrotransposon Ty, Polyoma virus; tobacco mosaic virus; Flock House Virus, Cowpea Chlorotic Mottle Virus; a Cowpea Mosaic Virus; and alfalfa mosaic virus.

Proper assembly of the fragment or mutant of recombinant protein or coat protein into a VLP may be tested, as one skilled in the art would appreciate by expressing the protein in E. coli, optionally purifying the capsids by gel filtration from cell lysate, and analysing the capsid formation in an immunodiffusion assay (Ouchterlony test) or by Electron Microscopy (EM) (Kozlovska, T. M.. et al, Gene 757:133-37 (1993)). Immunodiffusion assays and EM may be directly performed on cell lysate.

### c. Nanotransporters based on protein cages

In a further described variation, the nanotransporter is a protein cage. The term "protein cage", as used herein, relates to self-assembling macromolecular structure formed by one or more different proteins which are capable of forming a constrained internal environment. Protein cages can have different core sizes, ranging from 1 to 30 nm (e.g., the internal diameter of the shells). Preferred protein cages include ferritin protein cages, heat-shock protein cages as described in WO08124483 and the like.

### d. Nanotransporters based on nanoparticles

In another variation, the nanotransporters are nanoparticles. These include lipid nanoparticles as well as polymeric nanoparticles.

Polymeric nanoparticles are formed by a polymeric matrix which is attached to the adenoviral short fiber targeting moiety. Non-limiting examples of biocompatible polymers that may be useful in the polymeric nanoparticules according to the present invention include polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, or polyamines, polyglutamate, dextran, polyanhydrides, , polyurethanes, polymethacrylates, polyacrylates or polycyanoacrylates.polydioxanone (PDO), polyhydroxyalkanoate, polyhydroxybutyrate, poly(glycerol sebacate), polyglycolide, polylactide, PLGA, polycaprolactone or combinations thereof.

Alternatively, the nanoparticles may be lipid nanoparticles such as a liposome or a micelle. Formation of micelles and liposomes from, for example, vesicle- forming lipids, is known in the art. Vesicle-forming lipids refer to lipids that spontaneously form lipid bilayers above their gel-to-liquid crystalline phase transition temperature range. Such lipids typically have certain features that permit spontaneous bilayer formation, such as close to identical cross-section areas of their hydrophobic and hydrophilic portions permitting packing into lamellar phases. Lipids capable of stable incorporation into lipid bilayers, such as cholesterol and its various analogs, can be incorporated into the lipid bilayer during bilayer formation. The vesicle-forming lipids are preferably lipids having two hydrocarbon chains, typically acyl chains, and a head group, either polar or nonpolar. There are a variety of synthetic vesicle-forming lipids and naturally-occurring vesicle-forming lipids, including the phospholipids, such as phosphatidylcholine, phosphatidylethanolamine, phosphatidic acid, phosphatidylinositol, and sphingomyelin, where the two-hydrocarbon chains are typically between about 14- 22 carbon atoms in length, and either saturated or having varying degrees of unsaturation. The above-described lipids and phospholipids whose acyl chains have varying degrees of saturation can be obtained commercially or prepared according to published methods. Other suitable lipids include phospholipids, sphingolipids, glycolipids, and sterols, such as cholesterol.

Polymeric and lipidic nanotransporters can additionally include a coating of a amphiphilic compound that surrounds the polymeric material forming a shell for the particle or a stealth material that can allow the particles to evade recognition by immune system components and increase particle circulation half life. The amphiphilic compound can be, but is not limited to, one or a plurality of the following: naturally derived lipids, surfactants, or synthesized compounds with both hydrophilic and hydrophobic moieties. The water miscible solvent can be, but is not limited to: acetone, ethanol, methanol, and isopropyl alcohol. Separately, a biodegradable polymeric material is mixed with the agent or agents to be encapsulated in a water miscible or partially water miscible organic solvent. In a preferred embodiment, the biodegradable polymer can be any of the biodegradable polymers disclosed herein, for example, poly(D,L-lactic acid), poly(D,L-glycolic acid), poly(epsilon-caprolactone), or their copolymers at various molar ratios. For example, the nanotransporters can include a polymer- polyethylene glycol or a lipid-polyethylene glycol conjugate, to provide an external surface coating of polymer chains. The polymer polyethylene glycol is exemplary, and other polymers are suitable and are contemplated. Preparation of a vesicle-forming lipid derived with a hydrophilic polymer to form a lipopolymer is described, for example in U.S. Pat. No. 5,013,556. It will also be appreciated that the polymer or lipidic nanodevice can be formed from multiple layers of the same or different material.

### B. THE SUBGROUP F ADENOVIRAL SHORT FIBER PROTEIN

The nanotransporters of the invention contain as targeting moiety the short fiber protein of a subgroup F adenovirus The expression "subgroup F adenovirus", as used herein, relates to a group of viruses also known as enteric adenoviruses characterized in that, in contrast to other adenoviruses, do not rely on the interaction between RGD motifs in the fiber protein and the integrin receptor on the surface of the target cell for cell entry. Instead, the subgroup F adenoviruses may use an alternative mechanism for facilitating endocytosis. In contrast to all other human adenovirus species, enteric adenoviruses are also characterized in that they possess two different fiber proteins, both of which are found in each virion in approximately equal numbers. There is a long fiber, which has been shown to bind the coxsackie B virus and adenovirus receptor (CAR) and, additionally, a short fiber that does not bind CAR, and is of unknown function. It is possible that these two unique properties of enteric adenoviruses, namely the absence of an RGD motif in the penton base and the unusual presence of a second fiber are related to the special ability of these viruses to infect cells in the human gastric system. Further characteristics of enteric adenoviruses are the relatively high isoelectric point of their structural proteins, increased viability after exposure to an acidic environment (as found in parts of the gastric tract), and their ability to bind certain lipids commonly found in gastric mucosa, especially after exposure to low pH. The invention may be carried out using the short fiber protein of Ad40 (SEQ ID NO:1) or of the Ad41 (SEQ ID NO:2) serotypes. In a preferred embodiment, the short fiber protein of a group F adenovirus is the short fibre protein of Ad40.

In the present invention, the term "at least a part of the short fiber protein of a subgroup F adenovirus" refers to any of the domains of the short fiber protein of a subgroup F adenovirus which preserve the targeting functionalilty of the complete protein. Suitable fragments of the short fibre protein include at least the knob domain of a short fiber protein of group F adenovirus. The Ad40 tail domain corresponds to aminoacids 1 to 42 of SEQ ID No:1, the Ad40 shaft domain corresponds to aminoacids 43 to 233 from SEQ ID NO:1 and the Ad40 knob domain corresponds to aminoacids 234 to 387 from SEQ ID NO:1. The Ad41 tail domain corresponds to aminoacids 1 to 42 of SEQ ID No:2, the Ad41 shaft domain corresponds to aminoacids 43 to 233 from SEQ ID NO:2 and the Ad41 knob domain corresponds to aminoacids 234 to 387 from SEQ ID NO:2.

The skilled person will appreciate that the nanotransporters of the invention may also contain as targeting moieties hybrid fiber proteins which comprise the region of the adenovirus subgroup F short fiber protein which is able to target the nanotransporter to CD14+ cells and a region of a different adenovirus subgroup. In a preferred embodiment, said different adenovirus group is an adenovirus of subgroup C and, more preferably, an adenovirus 1, 2, 5 or 6. Thus, the fiber protein used in the nanotransporters of the invention may be formed by a fusion protein of the knob domain of the short fiber of a subgroup F adenovirus (Ad40 or Ad41 and the shaft and tail regions of a subgroup F adenovirus ( for instance, Ad5). In another embodiment, the fiber protein used in the nanotransporters of the invention may be formed by a fusion protein of the knob and shaft domain of the short fiber of a subgroup F adenovirus (Ad40 or Ad41) and the tail regions of a subgroup F adenovirus ( for instance, Ad5). In a still more preferred embodiment, the short fiber protein contains the tail, shaft and knob region of a short fiber protein of a subgroup F, more preferably, of Ad40 or Ad41.

The present disclosure further refers to functionally equivalent variants of the subgroup F adenovirus short fiber protein. The term "functionally equivalent variant", as used herein, refesr to polypeptides resulting from the addition, deletion or substitution of one or more amino acids with respect to the native short fiber protein and which maintains substantially the biological activity of the short fiber protein. The variant can have "conservative" changes, wherein a substituted amino acid has similar structural or chemical properties, e.g., replacement of leucine with isoleucine. Alternatively, a variant can have "nonconservative" changes, e.g., replacement of a glycine with a tryptophan. Analogous minor variations can also include amino acid deletion or insertion, or both. The variants of the subgroup F adenovirus fiber protein preferably have a sequence identity with the adenovirus fiber protein of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity between the variants and the natural ligand is determined by using computer algorithms and methods that are widely known for the persons skilled in the art. The identity between two amino acid sequences is determined by using the BLASTP algorithm (BLASTManual, Altschul, S., et al, NCBI NLM NIH Bethesda, Md. 20894, Altschul, S., et al., J. Mol. Biol. 21 5: 403-410 (1990) though other similar algorithms can also be used.

Suitable assays for determining whether a given variant of the adenovirus subgroup F short fiber protein is a functionally equivalent thereof is, for instance, the method as described in example 4 of the present invention based on the ability of the short fiber protein variant to diret a chimeric adenovirus which fiber protein is the short fiber protein variant to a CD14+ cell (e.g. a monocyte or a macrophage).

The short fiber protein acts as a targeting moiety for the nanotransporter and thus, must be present in the outer surface of the nanotransporter. The short fiber protein can be coupled to the nanotransporter once it has been formed or, alternatively, it can be incorporated into the sequence of the nanotransporter units so that, upon assembly of the nanotransporter, it will appear on the outer surface.

In the case wherein the short fiber protein as added to the preformed nanotransporter, this may be done by adding functional groups to the nanotransporter for subsequent attachment to additional moieties. Preferred functional groups for attachment are amino groups, carboxy groups, oxo groups and thiol groups. These functional groups can then be attached, either directly or indirectly through the use of a linker. Linkers are well known in the art; for example, homo-or hetero-bifunctional linkers as are well known (see 1994 Pierce Chemical Company catalog, technical section on cross-linkers, pages 155-200, as well as the 2003 catalog, both of which are incorporated herein by reference). Preferred linkers include, but are not limited to, alkyl groups (including substituted alkyl groups and alkyl groups containing heteroatom moieties), with short alkyl groups, esters, amide, amine, epoxy groups and ethylene glycol and derivatives being preferred, with propyl, acetylene, and C2 alkene being especially preferred.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the "-amino groups of lysine, arginine, and histidine side chains, acetylation of the N-terminal amine, and amidation of any C-terminal carboxyl group.

Another type of covalent modification of the nanotransporters, if appropriate, comprises altering the native glycosylation pattern of the polypeptides forming said nanotransporter. The expression "altering the native glycosylation pattern" is intended for purposes herein to mean deleting one or more carbohydrate moieties found in native sequence of the cage monomer, and/or adding one or more glycosylation sites that are not present in the native sequence.

Addition of glycosylation sites to cage polypeptides may be accomplished by altering the amino acid sequence thereof. The alteration may be made, for example, by the addition of, or substitution by, one or more serine or threonine residues to the native sequence polypeptide (for O-linked glycosylation sites). The amino acid sequence may optionally be altered through changes at the DNA level, particularly by mutating the DNA encoding the polypeptide at preselected bases such that codons are generated that will translate into the desired amino acids. Another means of increasing the number of carbohydrate moieties on the polypeptide is by chemical or enzymatic coupling of glycosides to the polypeptide. Such methods are described in the art, e. g., in WO 87/05330 published 11 September 1987, and in Aplin and Wriston, CRC Crit. Rev. Biochem., pp. 259-306 (1981).

Removal of carbohydrate moieties present on the polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophvs., 259: 52 (1987) and by Edge et al., Anal. Biochem., 118: 131 (1981). Enzymatic cleavage of carbohydrate moieties on polypeptides can be achieved by the use of a variety of endo-and exo-glycosidases as described by Thotakura et al., Meth. Enzymol., 138: 350 (1987).

Another type of covalent modification of nanotransporter moieties comprises linking the polypeptide to one of a variety of nonproteinaceous polymers, e. g., polyethylene glycol, polypropylene glycol, or polyoxyalkylenes, in the manner set forth in U. S. Patent Nos. 4,640, 835; 4,496, 689; 4,301, 144; 4,670, 417; 4,791, 192 or 4,179, 337.

This finds particular use in increasing the physiological half-life of the composition.

In those embodiments where the nanotransporter is a lipidic nanotransporter, attachment of the short fiber protein is carried out through chemical means, such as reacting activated lipids such as PE-malimide to activate free amines of an antibody with agents such as Traut's Reagent. Lipid conjugated short fiber protein can then be incorporated into the lipid nanoparticle.

The polypeptides forming the nanotransporters may also be modified so as to create chimeric molecules comprising part of all of the nanotransporter protein and the short fiber protein or variant thereof. This is usually carried out by fusing the short fiber protein to the N- or C-terminal end of the polypeptide which is capable of forming nanotransporter. Alternatively, the short fiber protein may be placed within an internal loop of the nanotransported constituent. Preferably, the internal loop is chosen so that, upon assembly of the nanotransporter, the short fiber protein is exposed on the outer surface of the nanotransporter. In one embodiment, such a chimeric molecule comprises a fusion of a nanotransporter polypeptide (viral capsid protein, VLP component, protein cage component and the like) with a tag polypeptide which provides an epitope to which an anti-tag antibody can selectively bind. The epitope tag is generally placed at the amino-or carboxyl-terminus of the polypeptide. The presence of such epitope-tagged forms of a cage polypeptide can be detected using an antibody against the tag polypeptide. Also, provision of the epitope tag enables the cage polypeptide to be readily purified by affinity purification using an anti-tag antibody or another type of affinity matrix that binds to the epitope tag.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8: 2159-2165 (1988)] ; the c-myc tag and the 8F9,3C7, 6E10, G4, B7 and 9E10 antibodies thereto [Evan et al., Molecular and Cellular Biology, 5 : 3610-3616 (1985) ] ; and the Herpes Simplex virus glycoprotein D (gD) tag and its antibody [Paborsky et al., Protein Engineering, 3 (6): 547-553 (1990)]. Other tag polypeptides include the Flag-peptide [Hopp et al., BioTechnologv, 6 : 1204-1210 (1988) ] ; the KT3 epitope peptide [Martin et al., Science, 255: 192-194 (1992) ]; tubulin epitope peptide [Skinner et al., J. Biol. Chem., 266: 15163-15166 (1991)] ; and the T7 gene 10 protein peptide tag [Lutz-Freyermuth et al., Proc. Natl. Acad. Sci. USA, 87: 6393-6397 (1990)].

The number of short fiber proteins on each nanotransporter is not limiting in the present invention as long as an efficient targeting of the nanotransporters to the target CD14+ cells is achieved. Accordingly, the nanotransporter may comprise from as low as 1 short fiber protein to as many as several thousands. It will be appreciated that, depending on the method used for the preparation of the nanotransporters, it will be possible to adjust the number of short fiber protein. Thus, if the short fiber protein-modified nanotransporters are obtained by chemical coupling of the short fiber protein to an activated nanotransporter, it will be possible to obtain a variable number of short fiber protein on the surface of the nanotransporter depending on the ratio of short fiber protein to nanotransporter used during the coupling reaction. Wherein the short fiber protein is attached to the nanotransported via specific interactions between binding pairs present in the short fiber protein and the nanotransporter, it will be possible to adjust the number of the short fiber proteins on the nanotransporter by adding a variable number of binding pairs onto the monomers forming the nanotransporter.

### C. IN VITRO USES OF THE NANOTRANSPORTERS OF THE INVENTION

The nanotransporters of the invention, by virtue of the presence within their surface of the adenoviral short fiber protein, show a high and selective affinity towards cells of the monocyte-macrophage lineage and, in particular, towards CD14⁺ mononuclear cells. Thus, the nanotransporters are particularly suited for delivering the contents of the nanotransporters to cells of the monocyte-macrophage lineage mononuclear cells. Thus, described herein is an *in vitro* method for delivering a compound of interest to a cell of the monocyte-macrophage lineage cell which comprises contacting said cell with a nanotransporter carrying said compound of interest and wherein the transporter contains at least a part of the short fiber protein of a group F adenovirus or a functionally equivalent variant thereof.

In a first step, the *in vitro* method comprises contacting the nanotransporter with a cell of the monocyte-macrophage lineage.

The expression "a cell of the monocyte-macrophage lineage", as used herein, relates to a CD14+ positive cell derived from monoblasts.

The term "monoblast", as used herein, refers to a type of hematopietic stem cells from about 12 to 20 µm in diameter that has a nuclear to cytoplasm ration of 4:1 to 3:1, and, like most myeloid blasts, has a round to oval nucleus with fine chromatin structure. One to four nucleoli are usually visible. The monoblast is the first stage of monocyte-macrophage maturation. The developmental stages of the monoblast are: CFU-GM (pluripotential hemopoietic stem cell or hemocytoblast), monoblast, promonocyte, monocyte and macrophage. CD14+ cells derived from monoblasts include, without limitation, monocytes, macrophages and myeloid dendritic cells.

The term "monocyte", as used herein, refers to a type of white blood cells that have two main functions in the immune system: (1) replenish resident macrophages and dendritic cells under normal states, and (2) in response to inflammation signals, monocytes can move quickly (approx. 8-12 hours) to sites of infection in the tissues and divide/differentiate into macrophages and dendritic cells to elicit an immune response. Half of them are stored in the spleen. Monocytes are usually identified in stained smears by their large bilobate nucleus. In addition to the expression of CD14, monocytes also show expression of one or more of the following surface markers 125I-WVH-1, 63D3, Adipophilin, CB12, CD11a, CD11b, CD15, CD54, Cd163, cytidine deaminase, Flt-1, and the like.

The term monocyte includes, without limitation both the classical monocyte and the non-classical pro-inflammatory monocyte, which are both present in human blood.

The term "the classical monocyte",as used herein, refers to a type of monocyte cell characterized by high level expression of the CD14 cell surface receptor (CD14++ monocyte) and the term "the non-classical pro-inflammatory monocyte", as used herein, refers to a cell with low level expression of CD14 and with additional co-expression of the CD16 receptor (CD14+CD16+ monocyte) which are characterized by producing high amounts of pro-inflammatory cytokines such as tumor necrosis factor and interleukin-12 in response to stimulation by microbial products. These cells develop from the CD14++ monocytes

The term "macrophage", as used herein, refers to CD14+ positive cells derived from the differentiation of the monocytes characterized in that are phagocytes, acting in both non-specific defense (innate immunity) as well as to help initiate specific defense mechanisms (adaptive immunity) of vertebrate animals. Their role is to phagocytose (engulf and then digest) cellular debris and pathogens either as stationary or as mobile cells, and to stimulate lymphocytes and other immune cells to respond to the pathogen.

In addition to the expression of CD14, macrophages also show expression of one or more of the following surface markers: CD11b, F4/80(mice)/EMR1(human), Lysozyme M, MAC-1/MAC-3, 27E10, Carboxypeptidase M, Cathepsin K, CD163 and CD68. These markers can be determined by flow cytometry or immunohistochemical staining.

The term "myeloid dendritic cell", as used herein, refers to a population of dendritic cells which derive from monocytes and which include, without limitation, mDC-1 and mDC-2. In addition to the expression of CD14, myeloid dendritic cells also show expression of one or more of the following surface markers: ADAM19, BDCA-2, CDa, CD11c CD21, CD86, CD208, Clusterin, Estrogen Receptor-alpha.

The contacting step can be carried out using standard methods known in the art and will depend on the type of cells being selected. The method contemplates the use of any suitable nanotransporter as defined in the previous section. Thus, the cell of the monocyte-macrophage lineage may be contacted with a virus, a VLP, a protein cage or a nanoparticle provided that the nanotransported is modified with the adenoviral short fiber protein. In a preferred embodiment, the nanotransproter is an adenoviral and, more preferably, a serotype 5 adenovirus. Moreover, the nanotransporter may contain any adenovirus subgroup F short fiber protein as previously defined. In a preferred embodiment, the short fiber protein is the short fiber protein of Ad40.

The nanotransporters can be used for delivering a product of interest to cells of the monocyte-macrophage lineage and, in particular, to blood mononuclear cells expressing CD14+ such as monocytes and macrophages. The in vitro method according to the invention is also suitable for delivering a compound of interest to monocytic or macrophage-like established cell lines such as THP-1, U-937 and RAW 264.7. Moreover, the in vitro method of the invention can also be used for delivering a compound of interest to monocyte-derived dendritic cells.

### D. DIAGNOSTIC USES OF THE NANOTRANSPORTERS OF THE INVENTION

The nanotransporters described herein can also be used for delivering imaging agents to cells of the monocyte-macrophage lineage, thus facilitating their subsequent identification and quantification in biological samples. In a described variation, the nanotransporters are used for the detection of CD14+ mononuclear cells since altered monocyte counts are usually associated with different diseases such as chronic inflammation, stress response, hyperadrenocorticism, immune-mediated disease, infectious mononucleosis, pyogranulomatous disease, necrosis, red cell regeneration and viral fever. Thus, in another aspect, described herein is a method for the diagnosis of a diseases associated with an abnormal count of blood mononuclear CD14+ cells which comprises contacting the blood mononuclear CD14+ cell with a nanotransporter of the invention previously loaded with a detectable compound and detecting the blood mononuclear CD14+ cells by tracing the detectable compound.

The diagnostic methods using the nanotransporters usually involves isolating blood cells from the patient, contacting the blood cells with an imaging agent-loaded nanotransporter according to the invention and quantifying the percentage of blood cells wherein the imaging agent is detected. In another variation, the imaging or diagnostic conjugates can be administered to the patient as a diagnostic composition comprising a conjugate and a pharmaceutically acceptable carrier and thereafter blood cells can be collected from the patient to quantify the percentage of monocytes. In this embodiment, the composition is typically formulated for parenteral administration and is administered to the patient in an amount effective to enable imaging of monocytes. Suitable imaging agents for use in the diagnostic methods include radiolabeled compounds, (e.g. technetium, gallium, indium, and a positron emitting radionucleides or PET imaging agent) and chromophores such as, for example, fluorescein, rhodamine, Texas Red, phycoerythrin, Oregon Green, AlexaFluor 488 (Molecular Probes, Eugene, Oreg.), Cy3, Cy5, Cy7, and the like.

Diagnosis typically occurs before treatment. However, in the diagnostic methods described herein, the term "diagnosis" can also mean monitoring of the disease state before, during, or after treatment to determine the progression of the disease state. The monitoring can occur before, during, or after treatment, or combinations thereof, to determine the efficacy of therapy, or to predict future episodes of disease. The imaging can be performed by any suitable imaging method known in the art, such as intravital imaging.

The diagnostic method contemplates the use of any suitable nanotransporter as defined in the previous section. Thus, the cell of the monocyte-macrophage lineage may be contacted with a virus, a VLP, a protein cage or a nanoparticle provided that the nanotransported is modified with the adenoviral short fiber protein. In a preferred embodiment, the nanotransproter is an adenoviral and, more preferably, a serotype 5 adenovirus. Moreover, the nanotransporter may contain any adenovirus subgroup F short fiber protein as previously defined. In a preferred embodiment, the short fiber protein is the short fiber protein of Ad40.

### E. MEDICAL USES OF THE NANOTRANSPORTERS OF THE INVENTION

The efficient and specific interaction of the nanotransporters with cells of the monocyte-macrophage lineage allows the use of said nanotransporters for the treatment of any disease wherein it is desirable to deliver a compound of interest to cells of the monocyte-macrophage lineage. Thus, in another aspect, described herein is a nanotransporter comprising a product of interest and at least a part of the short fiber protein of a subgroup F adenovirus or a functionally equivalent variant thereof for use in medicine.

According to the present invention, a "product of interest" as used herein, refers to any agent ("payload"), including, for example, therapeutic agents (e.g. anti-cancer agents), diagnostic agents (e.g. contrast agents; radionuclides; and fluorescent, luminescent, and magnetic moieties), prophylactic agents (e.g. vaccines), and/or nutraceutical agents (e.g. vitamins, minerals, etc.) may be delivered by the nanotransporters of the invention. Exemplary agents to be delivered in accordance with the present invention include, but are not limited to, small molecules (e.g. cytotoxic agents), nucleic acids (e.g., cDNAs, siRNA, RNAi, and microRNA agents), proteins (e.g. antibodies), peptides, lipids, carbohydrates, hormones, metals, radioactive elements and compounds, drugs, vaccines, immunological agents, etc., and/or combinations thereof. In some embodiments, the agent to be delivered is an agent useful in the treatment of cancer (e.g., prostate cancer). In a particular embodiment, the drug or other payload is released in a controlled release manner from the particle and allowed to interact locally with the particular targeting site (e.g., a tumor). The term "controlled release" (and variants of that term) as used herein (e.g., in the context of "controlled-release system") is generally meant to encompass release of a substance (e.g., a drug or a protein) at a selected site or otherwise controllable in rate, interval, and/or amount. Controlled release encompasses, but is not necessarily limited to, substantially continuous delivery, patterned delivery (e.g., intermittent delivery over a period of time that is interrupted by regular or irregular time intervals), and delivery of a bolus of a selected substance (e.g., as a predetermined, discrete amount if a substance over a relatively short period of time (e.g., a few seconds or minutes). For example, a targeting portion may cause the particles to become localized to a tumor, a disease site, a tissue, an organ, a type of cell, etc. within the body of a subject, depending on the targeting moiety used. For example, a targeting moiety, e.g., an aptamer, may become localized to prostate cancer cells.

In particular embodiments, the agent to be delivered is a nucleic acid selected from the group consisting of natural or unnatural siRNAs, shRNAs, microRNAs, ribozymes, DNA plasmids, aptamers, antisense oligonucleotides, randomized oligonucleotides, or ribozymes.

As used herein, the term "pharmaceutically acceptable carrier" means a non- toxic, inert solid, semi-solid or liquid filler, diluent, encapsulating material or formulation auxiliary of any type. Remington's Pharmaceutical Sciences. Ed. by Gennaro, Mack Publishing, Easton, Pa., 1995 discloses various carriers used in formulating pharmaceutical compositions and known techniques for the preparation thereof. Some examples of materials which can serve as pharmaceutically acceptable carriers include, but are not limited to, sugars such as lactose, glucose, and sucrose; starches such as com starch and potato starch; cellulose and its derivatives such as sodium carboxymethyl cellulose, ethyl cellulose, and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols such as propylene glycol; esters such as ethyl oleate and ethyl laurate; agar; detergents such as TWEEN™ 80; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. If filtration or other terminal sterilization methods are not feasible, the formulations can be manufactured under aseptic conditions.

The pharmaceutical compositions of this invention can be administered to a patient by any means known in the art including oral and parenteral routes. According to such embodiments, inventive compositions may be administered by injection (e.g., intravenous, subcutaneous or intramuscular, intraperitoneal injection), rectally, vaginally, topically (as by powders, creams, ointments, or drops), or by inhalation (as by sprays).

In a particular embodiment, the nanotransporters of the present invention are administered to a subject in need thereof systemically, e.g., by IV infusion or injection. Injectable preparations, for example, sterile injectable aqueous or oleaginous suspensions may be formulated according to the known art using suitable dispersing or wetting agents and suspending agents. The sterile injectable preparation may also be a sterile injectable solution, suspension, or emulsion in a nontoxic parenterally acceptable diluent or solvent, for example, as a solution in 1,3-butanediol. Among the acceptable vehicles and solvents that may be employed are water, Ringer's solution, U.S.P., and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. In one embodiment, the inventive conjugate is suspended in a carrier fluid comprising 1 % (w/v) sodium carboxymethyl cellulose and 0.1% (v/v) TWEEN™ 80. The injectable formulations can be sterilized, for example, by filtration through a bacteria-retaining filter, or by incorporating sterilizing agents in the form of sterile solid compositions which can be dissolved or dispersed in sterile water or other sterile injectable medium prior to use.

Compositions for rectal or vaginal administration may be suppositories which can be prepared by mixing the inventive conjugate with suitable non-irritating excipients or carriers such as cocoa butter, polyethylene glycol, or a suppository wax which are solid at ambient temperature but liquid at body temperature and therefore melt in the rectum or vaginal cavity and release the inventive conjugate.

Dosage forms for topical or transdermal administration of an inventive pharmaceutical composition include ointments, pastes, creams, lotions, gels, powders, solutions, sprays, inhalants, or patches. The inventive conjugate is admixed under sterile conditions with a pharmaceutically acceptable carrier and any needed preservatives or buffers as may be required. Ophthalmic formulations, ear drops, and eye drops are also contemplated as being within the scope of this invention. The ointments, pastes, creams, and gels may contain, in addition to the inventive conjugates of this invention, excipients such as animal and vegetable fats, oils, waxes, paraffins, starch, tragacanth, cellulose derivatives, polyethylene glycols, silicones, bentonites, silicic acid, talc, and zinc oxide, or mixtures thereof. Transdermal patches have the added advantage of providing controlled delivery of a compound to the body. Such dosage forms can be made by dissolving or dispensing the inventive conjugates in a proper medium. Absorption enhancers can also be used to increase the flux of the compound across the skin. The rate can be controlled by either providing a rate controlling membrane or by dispersing the inventive conjugates in a polymer matrix or gel. Powders and sprays can contain, in addition to the inventive conjugates of this invention, excipients such as lactose, talc, silicic acid, aluminum hydroxide, calcium silicates, and polyamide powder, or mixtures thereof. Sprays can additionally contain customary propellants such as chlorofluorohydrocarbons. When administered orally, the inventive nanotransporter can be, but are not necessarily, encapsulated. A variety of suitable encapsulation systems are known in the art ("Microcapsules and Nanoparticles in Medicine and Pharmacy," Edited by Doubrow, M., CRC Press, Boca Raton, 1992; Mathiowitz and Langer J. Control. Release 5:13, 1987; Mathiowitz et al. Reactive Polymers 6:275, 1987; Mathiowitz et al. J. Appl. Polymer Sci. 35:755, 1988; Langer Ace. Chem. Res. 33:94,2000; Langer J. Control. Release 62:7,1999; Uhrich et al. Chem. Rev. 99:3181,1999; Zhou et al. J. Control. Release 75:27, 2001; and Hanes et al. Pharm. Biotechnol. 6:389,1995). The inventive conjugates may be encapsulated within biodegradable polymeric microspheres or liposomes. Examples of natural and synthetic polymers useful in the preparation of biodegradable microspheres include carbohydrates such as alginate, cellulose, polyhydroxyalkanoates, polyamides, polyphosphazenes, polypropylfumarates, polyethers, polyacetals, polycyanoacry lates, biodegradable polyurethanes, polycarbonates, polyanhydrides, polyhydroxyacids, poly(ortho esters), and other biodegradable polyesters. Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides.

Pharmaceutical compositions for oral administration can be liquid or solid. Liquid dosage forms suitable for oral administration of inventive compositions include pharmaceutically acceptable emulsions, microemulsions, solutions, suspensions, syrups, and elixirs. In addition to an encapsulated or unencapsulated conjugate, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants, wetting agents, emulsifying and suspending agents, sweetening, flavoring, and perfuming agents. As used herein, the term "adjuvant" refers to any compound which is a nonspecific modulator of the immune response. In certain embodiments, the adjuvant stimulates the immune response. Any adjuvant may be used in accordance with the present invention. A large number of adjuvant compounds is known in the art (Allison Dev. Biol. Stand. 92:3-11, 1998; Unkeless et al. Annu. Rev. Immunol. 6:251-281,1998; and Phillips et al. Vaccine 10:151-158,1992).

Solid dosage forms for oral administration include capsules, tablets, pills, powders, and granules. In such solid dosage forms, the encapsulated or unencapsulated conjugate is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar-agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. It will be appreciated that the exact dosage of the targeted reverse micelle particle is chosen by the individual physician in view of the patient to be treated, in general, dosage and administration are adjusted to provide an effective amount of the targeted particle to the patient being treated. As used herein, the "effective amount" of a targeted particle refers to the amount necessary to elicit the desired biological response. As will be appreciated by those of ordinary skill in this art, the effective amount of targeted particle may vary depending on such factors as the desired biological endpoint, the drug to be delivered, the target tissue, the route of administration, etc. For example, the effective amount of targeted particle containing an anti-cancer drug might be the amount that results in a reduction in tumor size by a desired amount over a desired period of time. Additional factors which may be taken into account include the severity of the disease state; age, weight and gender of the patient being treated; diet, time and frequency of administration; drug combinations; reaction sensitivities; and tolerance/response to therapy.

The nanotransporters of the invention may be formulated in dosage unit form for ease of administration and uniformity of dosage. The expression "dosage unit form" as used herein refers to a physically discrete unit of nanotransporter appropriate for the patient to be treated. It will be understood, however, that the total daily usage of the compositions of the present invention will be decided by the attending physician within the scope of sound medical judgment. For any nanotransporter, the therapeutically effective dose can be estimated initially either in cell culture assays or in animal models, usually mice, rabbits, dogs, or pigs. The animal model is also used to achieve a desirable concentration range and route of administration. Such information can then be used to determine useful doses and routes for administration in humans. Therapeutic efficacy and toxicity of nanotransporters can be determined by standard pharmaceutical procedures in cell cultures or experimental animals, e.g., ED50 (the dose is therapeutically effective in 50% of the population) and LD50 (the dose is lethal to 50% of the population). The dose ratio of toxic to therapeutic effects is the therapeutic index, and it can be expressed as the ratio, LD50/ED50. Pharmaceutical compositions which exhibit large therapeutic indices may be useful in some embodiments. The data obtained from cell culture assays and animal studies can be used in formulating a range of dosage for human use.

The nanotransporter can be used to deliver a compound of interest to undifferentiated and unstimulated monocytes, cells of the monocyte-macrophage lineage, which includes blood CD14+ mononuclear cell as well as other cell types derived from blood CD14+ cells as a result of spontaneous or induced maturation or differentiation processes and thus, is suitable for the treatment of diseases in which such cells are involved. Thus, also described herein is a nanotransporter comprising a product of interest and at least a part of the short fiber protein of a subgroup F adenovirus or a functionally equivalent variant thereof for use in the treatment of a disease associated with cells of the monocyte-macrophage lineage.

In another aspect, described is a method of treatment of a disease associated with cells of the monocyte-macrophage lineage comprising the administration to a subject in need thereof of a nanotransporter comprising a product of interest and at least a part of the short fiber protein of a subgroup F adenovirus or a functionally equivalent variant thereof.

Thus, in another aspect, described is the use of a nanotransporter comprising a product of interest and at least a part of the short fiber protein of a subgroup F adenovirus or a functionally equivalent variant thereof for the preparation of a pharmaceutical composition for the treatment of a disease associated with cells of the monocyte-macrophage lineage.

The term "disease associated with cells of the monocyte-macrophage lineage" relates to any disease caused or augmented by cells of the monocyte-macrophage lineage as well as to any disease caused by a deficient activity or reduced number of cells of the monocyte-macrophage lineage cells. In a preferred embodiment, the cell of the monocyte-macrophage lineage is a primary mononuclear blood cells or a monocyte, in which case the disease is selected from the group of:
(i) a disease characterized by an altered immune response
(ii) an inflammatory disease and
(iii) a disease characterized by an undesired proliferation of cell of the monocyte-macropahge lineage.

### Diseases characterized by an altered immune response

The expression "a disease characterized by an altered immune response" relates to diseases wherein the organism reacts abnormally towards exogenous infectious agents without triggering a full immune response. The expression "a disease characterized by an altered immune response" also includes diseases wherein the organism reacts abnormally towards endogenous components triggering an immune response leading to an auto-immune disease. Preferably the disease is an infectious disease or an allergic disease.

In the case wherein the immune response triggered by the organism towards and infectious agent is not sufficient, the nanotransporters of the invention can be used to deliver antigenic peptides from the infectious agent to blood CD14+ cells. The CD14+ cells will then differentiate to dendritic cells, thus acting as antigen presenting cells and presenting the antigenic peptide to the mediators of the humoral and cellular immune response. In an alternative embodiment, the nanotransporters of the invention may be used to deliver antigenic peptides to monocyte-derived dendritc cells which will then act as antigen-presenting cells to mediators of the humoral and cellular immune response. Thus, in one embodiment, the nanotransporters of the invention comprise antigenic peptides or nucleic acids encoding said antigenic peptides. Suitable antigenic peptides that can be used in the nanotransporters of the invention include but is not limited to, a viral antigen, a bacterial antigen, a fungal antigen, a differentiation antigen, a tumor antigen, an embryonic antigen, an antigen of oncogenes and mutated tumor-suppressor genes, a unique tumor antigen resulting from chromosomal translocations and/or derivatives thereof.

Viral antigens which are capable of eliciting an immune response against the virus include HIV-1 antigens, (such as tat, nef, gp120 or gp160, gp40, p24, gag, env, vif, vpr, vpu, rev), human herpes viruses, (such as gH, gL gM gB gC gK gE or gD or derivatives thereof or Immediate Early protein such as ICP27 , ICP47, ICP4, ICP36 from HSV1 or HSV2, cytomegalovirus, especially Human, (such as gB or derivatives thereof), Epstein Barr virus (such as gp350 or derivatives thereof), Varicella Zoster Virus (such as gpl, II, Ill and IE63), or from a hepatitis virus such as hepatitis B virus (for example Hepatitis B Surface antigen or Hepatitis core antigen), hepatitis C virus (for example core, E1, NS3 or NS5 antigens), from paramyxoviruses such as Respiratory Syncytial virus (such as F and G proteins or derivatives thereof), from parainfluenza virus, from rubella virus (such as proteins El and E2), measles virus, mumps virus, human papilloma viruses (for example HPV6, 11, 16, 18, eg LI, L2, E1, E2, E3, E4, E5, E6, E7), flaviviruses (e.g. Yellow Fever Virus, Dengue Virus, Tickborne encephalitis virus, Japanese Encephalitis Virus) or Influenza virus cells, such as HA, NP, NA, or M proteins, or combinations thereof), rotavirus antigens (such as VP7sc and other rotaviral components), and the like (see Fundamental Virology, Second Edition, eds. Fields, B. N. and Knipe, D. M. (Raven Press, New York, 1991) for additional examples of viral antigens)

Bacterial antigens include such as antigens from *Neisseria spp,* including *N. gonorrhea* and *N*. *meningitidis* (transferrin-binding proteins, lactoferrin binding proteins, PiIC and adhesins); antigens from *S*. *pyogenes* (such as M proteins or fragments thereof and C5A protease); antigens from *S. agalactiae*, *S. mutans*; *H. ducreyi*; *Moraxella spp, including M catarrhalis,* also known as *Branhamella* catarrhalis (such as high and low molecular weight adhesins and invasins); antigens from *Bordetella spp,* including *B. pertussis* (for example *parapertussis and B. bronchiseptica* (such as pertactin, pertussis toxin or derivatives thereof, filamenteous hemagglutinin, adenylate cyclase, fimbriae); antigens from *Mycobacterium spp., including M. tuberculosis, M. bovis, M. leprae, M. avium, M. paratuberculosis, M. smegmatis; Legionella spp, including L. pneumophila;* (for example ESAT6, Antigen 85A, -B or -C, MPT 44, MPT59, MPT45, HSPIO,HSP65, HSP70, HSP 75, HSP90, PPD 19kDa [Rv3763], PPD 38kDa [Rv0934] ); antigens from *Escherichia spp,* including *enterotoxic E. coli* (for example colonization factors, heat-labile toxin or derivatives thereof, heat-stable toxin or derivatives thereof), antigens from enterohemorragic *E. coli* and enteropathogenic *E. coli* (for example shiga toxin-like toxin or derivatives thereof); antigens from *Vibrio spp,* including *V. cholera* (for example cholera toxin or derivatives thereof); antigens from *Shigella spp, including S. sonnei, S. dysenteriae, S*. *flexnerii; Yersinia spp,* including Y. enterocolitica (for example a Yop protein); antigens from *Y. pestis, Y. pseudotuberculosis; Campylobacter spp,* including C. *jejuni* (for example toxins, adhesins and invasins); antigens from *Salmonella spp,* including *S. typhi, S. paratyphi, S. choleraesuis, S. enteritidis; Listeria spp.,* including *L. monocytogenes; Helicobacter spp,* including *H.* pylori (for example urease, catalase, vacuolating toxin); antigens from *Pseudomonas spp,* including *P. aeruginosa; Staphylococcus spp.,* including *S. aureus, S. epidermidis; Enterococcus spp.,* including *E. faecalis, E. faecium; Clostridium spp.,* including *C. tetani* (for example tetanus toxin and derivative thereof); antigens from *C. botulinum* (for example botulinum toxin and derivative thereof), antigens from *C. difficile* (for example clostridium toxins A or B and derivatives thereof); antigens from *Bacillus spp.,* including *B. anthracis* (for example anthrax toxin and derivatives thereof); *Corynebacterium spp.,* including C. *diphtheriae* (for example diphtheria toxin and derivatives thereof); antigens from *Borrelia spp.,* including *B. burgdorferi* (for example OspA, OspC, DbpA, DbpB); antigens from *B. garinii* (for example OspA, OspC, DbpA, DbpB), *B. afzelii* (for example OspA, OspC, DbpA, DbpB), antigens from *B. andersonfi* (for example OspA, OspC, DbpA, DbpB), antigens from *B. hermsii; Ehrlichia spp.,* including *E. equi* and the agent of the Human Granulocytic Ehrlichiosis; *Rickettsia spp,* including *R. rickettsii; Chlamydia spp.,* including C. *trachomatis* (for example MOMP, heparin-binding proteins); antigens from *Chlamydia pneumoniae* (for example MOMP, heparin-binding proteins), antigens from *C. psittaci; Leptospira spp.,* including *L. interrogans; Treponema spp.,* including *T. pallidum* (for example the rare outer membrane proteins), antigens from *T. denticola, T. hyodysenteriae;* antigens from *Plasmodium spp.,* including *P. falciparum; Toxoplasma spp.* and *T. gondii* (for example SAG2, SAGS, Tg34); antigens from *Entamoeba spp.,* including *E. histolytica; Babesia spp.,* including *B. microti; Trypanosoma spp.,* including T. *cruzi; Giardia spp.,* including *G. lamblia; leishmania spp.,* including *L. major; Pneumocystis spp.,* including *P. carinii; Trichomonas spp.,* including T. *vaginalis; Schisostoma spp.,* including *S. mansoni,* or derived from yeast such as *Candida spp.,* including C. *albicans; Cryptococcus spp.,* including C. *neoformans;* antigens from *M. tuberculosis* (such as Rv2557, Rv2558, RPFs: Rv0837c, Rv1884c, Rv2389c, Rv2450, Rv1009, aceA (Rv0467), PstS1 (Rv0932), SodA (Rv3846), Rv2031c 16kDal., Tb Ra12, Tb H9, Tb Ra35, Tb38-1, Erd 14, DPV, MTI, MSL, mTTC2 and hTCC1); antigens from Chlamydia (such as the High Molecular Weight Protein (HWMP), ORF3 (EP 366 412), and putative membrane proteins (Pmps); antigens from *Streptococcus spp,* including *S. pneumoniae* (PsaA, PspA, streptolysin, choline-binding proteins, the protein antigen Pneumolysin, and mutant detoxified derivatives thereof); antigens derived from *Haemophilus spp., including H. influenzae type B* (for example PRP and conjugates thereof); antigens from non typeable *H. influenzae* (such as OMP26, high molecular weight adhesins, P5, P6, protein D and lipoprotein D, and fimbrin and fimbrin derived peptides, or multiple copy variants or fusion proteins thereof); antigens derived from *Plasmodium falciparum* (such as RTS.S, TRAP, MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.)

Fungal antigens for use in the nanotransporters of the invention include, without limitation, e.g., Candida fungal antigen components; histoplasma fungal antigens such as heat shock protein 60 (HSP60) and other histoplasma fungal antigen components; cryptococcal fungal antigens such as capsular polysaccharides and other cryptococcal fungal antigen components; coccidiodes fungal antigens such as spherule antigens and other coccidiodes fungal antigen components; and tinea fungal antigens such as trichophytin and other coccidiodes fungal antigen components.

Protozoal antigens include, but are not limited to, *Plasmodium falciparum* antigens such as merozoite surface antigens, sporozoite surface antigens, circumsporozoite antigens, gametocyte/gamete surface antigens, blood-stage antigen pf, 55/RESA and other plasmodial antigen components; toxoplasma antigens such as SAG-1, p30 and other toxoplasmal antigen components; schistosomae antigens such as glutathione- S- transferase, paramyosin, and other schistosomal antigen components; leishmania major and other leishmaniae antigens such as gp63, lipophosphoglycan and its associated protein and other leishmanial antigen components; and *Trypanosoma cruzi* antigens such as the 75-77 kDa antigen, the 56 kDa antigen and other trypanosomal antigen components.

The antigen can be an allergen or environmental antigen, such as, but not limited to, an antigen derived from naturally occurring allergens such as pollen allergens (tree-, herb, weed-, and grass pollen allergens), insect allergens (inhalant, saliva and venom allergens), animal hair and dandruff allergens, and food allergens. Important pollen allergens from trees, grasses and herbs originate from the taxonomic orders of Fagales, Oleales, Pinoles and platanaceae including La. birch (Betula), alder (Alnus), hazel (Corylus), hornbeam (Carpinus) and olive (Olea), cedar (Cryptomeriaand Juniperus), Plane tree (Platanus), the order of Poales including i.e. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria. Other allergen antigens that may be used include allergens from house dust mites of the genus Dermatophagoides and Euroglyphus, storage mite e.g Lepidoglyphys, Glycyphagus and Tyrophagus, those from cockroaches, midges and fleas e.g. Blatella, Periplaneta, Chironomus and Ctenocepphalides, those from mammals such as cat, dog and horse, birds, venom allergens including such originating from stinging or biting insects such as those from the taxonomic order of Hymenoptera including bees (superfamily Apidae), wasps and ants (superfamily Formicoidae). Still other allergen antigens that may be used include inhalation allergens from fungi such as from the genera Alternaria and Cladosporium.

The antigen can also be a tumor antigens such as MAGE, MART-1/Melan-A, gp100, Dipeptidyl peptidase IV (DPPIV), adenosine deaminase-binding protein (ADAbp), cyclophilin b, Colorectal associated antigen (CRC)-0017-1A/GA733, Carcinoembryonic Antigen (CEA) and its antigenic epitopes CAP-1 and CAP-2, etv6, aml1, Prostate Specific Antigen (PSA) and its antigenic epitopes PSA-1, PSA-2, and PSA-3, prostate-specific membrane antigen (PSMA), T-cell receptor/CD3- 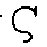 chain, MAGE-family of tumor antigens (e.g., MAGE-A1, MAGE-A2, MAGE-A3, MAGEA4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGEC5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p2lras, RCAS1, α-fetoprotein, E-cadherin, α-catenin,13-catenin, γ-catenin, pl20ctn, gp100^{Pme1117}, PRAME, NY-ESO-1, cdc27, adenomatous polyposis coli protein (APC), fodrin, Connexin 37, Ig- idiotype, p15, gp75, GM2 and GD2 gangliosides, viral products such as human papilloma virus proteins, Smad family of tumor antigens, lmp-1, P1A, EBV-encoded nuclear antigen (EBNA)-1, brain glycogen phosphorylase, SSX-1, SSX-2 (HOM-MEL40), SSX-3, SSX-4, SSX-5, SCP-1 and CT-7, and c-erbB-2, acute lymphoblastic leukemia (etv6, amll, cyclophilin b), B cell lymphoma (Ig-idiotype), glioma (E-cadherin, α-catenin,13-catenin, 7-catenin, p120ctn), bladder cancer (p2lras), biliary cancer (p2lras), breast cancer (MUC family, HER2/neu, c-erbB-2), cervical carcinoma (p53, p2lras), colon carcinoma (p2lras, HER2/neu, c-erbB-2, MUC family), colorectal cancer (Colorectal associated antigen (CRC)-0017-1A/GA733, APC), choriocarcinoma (CEA), epithelial cell cancer (cyclophilin b), gastric cancer (HER2/neu, c-erbB-2, ga733 glycoprotein), hepatocellular cancer, Hodgkins lymphoma (lmp-1, EBNA-1), lung cancer (CEA, MAGE-3, NY-ESO-1), lymphoid cell-derived leukemia (cyclophilin b), melanoma (p15 protein, gp75, oncofetal antigen, GM2 and GD2 gangliosides, MelanA/MART-1, cdc27, MAGE-3, p2lras, gp100^{Pme1117}), myeloma (MUC family, p21ras), non-small cell lung carcinoma (HER2/neu, c-erbB-2), nasopharyngeal cancer (lmp-1, EBNA-1), ovarian cancer (MUC family, HER2/neu, c-erbB-2), prostate cancer (Prostate Specific Antigen (PSA) and its antigenic epitopes PSA-1, PSA-2, and PSA-3, PSMA, HER2/neu, c-erbB-2, ga733 glycoprotein), renal cancer (HER2/neu, c-erbB-2), squamous cell cancers of the cervix and esophagus (viral products such as human papilloma virus proteins), testicular cancer (NY-ES0-1), and T cell leukemia (HTLV-1 epitopes).

The skilled person will appreciate that the antigen-loaded nanotransporters of the invention can be used by direct administration to a patient in need thereof or, alternatively, they can also be used for ex vivo therapy. For this purpose, monocytes can be isolated from the patient, contacted with the nanotransproters of the invention and either directly administered back to the patient or, alternatively, allowed to differentiate into dendritic cells which will then be delivered to the patient. Methods for promoting in vitro differentiation of monocytes to dendritic cells are known in the art (e.g. using a combination of GM-CSF and IL-4 or using methods such as those described in WO06012359).

In another aspect, the nanotransporters of the invention can be used for the treatment of diseases characterised by an increased immune response towards endogenous celular components (i.e. autoimmune diseases). Exemplary disease states include fibromyalgia, rheumatoid arthritis, osteoarthritis, ulcerative colitis, Crohn's disease, psoriasis, osteomyelitis, multiple sclerosis, atherosclerosis, pulmonary fibrosis, sarcoidosis, systemic sclerosis, organ transplant rejection (GVHD), lupus erythematosus, Sjogren's syndrome, glomerulonephritis, inflammations of the skin (e.g., psoriasis), and chronic inflammations. For this purpose, the nanotransporters are loaded with cytotoxic components that will lead to a reduction in the number of monocytes and thus, to a decreased in the number of dendritic cells. Exemplary cytotoxic compounds that can be used in the nanotransporters of the invention include clodronate, anthrax toxin, Pseudomonas exotoxin, typically modified so that these cytotoxic moieties do not bind to normal cells, and other toxins or cytotoxic agents including art-recognized chemotherapeutic agents such as adrenocorticoids, alkylating agents, antiandrogens, antiestrogens, androgens, estrogens, antimetabolites such as cytosine arabinoside, purine analogs, pyrimidine analogs, and methotrexate, busulfan, carboplatin, chlorambucil, cisplatin and other platinum compounds, tamoxiphen, taxol, cyclophosphamide, plant alkaloids, prednisone, hydroxyurea, teniposide, and bleomycin, nitrogen mustards, nitrosureas, vincristine, vinblastine, MEK kinase inhibitors, MAP kinase pathway inhibitors, PI-3-kinase inhibitors, mitochondrial perturbants, NFKB pathway inhibitors, proteosome inhibitors, pro-apoptotic agents, glucocorticoids, such as prednisolone, flumethasone, dexamethasone, and betamethasone, indomethacin, diclofenac, proteins such as pokeweed, saporin, momordin, and gelonin, non-steroidal anti-inflammatory drugs (NSAIDs), protein synthesis inhibitors, didemnin B, verrucarin A, geldanamycin, and the like.

### Inflammatory diseases

The monocytes are known to migrate to sites of inflammation in response to CCL2. Thus, the present invention contemplates the use of the nanotransporters of the invention to deliver anti-inflammatory agents to monocytes which will then be delivered to the sites of inflammation. Thus, the nanotransporters of the invention may be loaded with an anti-inflammatory agent and used to deliver said agent to a site of inflammation. Antiinflammatory agents that can be incorporated into the nanotransporters of the invention include, without limitation, steroidal anti-inflammatory agents, such as hydrocortisone, hydroxyltriamcinolone, alpha-methyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylester, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone acetonide, medrysone, amcinafel, amcinafide, betamethasone and the balance of its esters, chloroprednisone, chloroprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof. The nanotransporters may also contain nonsteroidal anti-inflammatory agents (NSAID), such as oxicams (piroxicam, isoxicam, tenoxicam, sudoxicam, and CP-14,304), salicylates (aspirin, disalcid, benorylate, trilisate, safapryn, solprin, diflunisal, and fendosal), acetic acid derivatives (diclofenac, fenclofenac, indomethacin, sulindac, tolmetin, isoxepac, furofenac, tiopinac, zidometacin, acematacin, fentiazac, zomepiract, clidanac, oxepinac, and felbinac), fenamates (mefenamic, meclofenamic, flufenamic, niflumic, and tolfenamic acids), propionic acid derivatives (ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic), pyrazoles (phenybutazone, oxyphenbutazone, feprazone, azapropazone, and trimethazone), arylacetic acid derivatives, arylbutyric acid derivatives, arylcarboxylic acids, arylpropionic acid derivatives, pyrazoles, pyrazolones, thiazinecarboxamides, eacetamidocaproic acid, S-adenosylmethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole, and tenidap. Inflammatory diseases that can be treated with the nanotransporters according to the invention include, without limitation, arthritis (including rheumatoid arthritis, juvenile rheumatoid arthritis, osteoarthritis, psoriatic arthritis, lupus-associated arthritis or ankylosing spondylitis), scleroderma, systemic lupus erythematosis, vasculitis, multiple sclerosis, autoimmune thyroiditis, dermatitis (including atopic dermatitis and eczematous dermatitis), autoimmune skin diseases, myasthenia gravis, inflammatory bowel disease (IBD), Crohn's disease, colitis, ulcerative colitis, diabetes mellitus (type I) and the like.

### Diseases associated with an increased proliferation/activity of cells of the monocyte-macrophage lineage

In another embodiment, the nanotransporters are suitable for the treatment associated with an increased proliferation or activity of a cell of the monocyte-macrophage lineage. These diseases include:
- hyperproliferative diseases of the monocyte/macrophage lineage
- diseases resulting from an increased interleukin-1 (IL-1) production by monocytes and
- diseases resulting from an increased TNF-α production by monocytes.

These diseases can be treated by reducing the number of monocytes using the nanotransporters of the invention for the delivery of cytotoxic compounds as defined above.

In one embodiment, the nanotransporters may be used for the treatment of disease associated with an undesired proliferation of cells of the monocyte-macropahge lineage such as acute or chronic monocytic leukemia, acute or chronic myelomonocytic leukemia and Juvenile myelomonocytic leukemia. Hyperproliferative diseses of the monocyte/macrophage lineage that can be treated with cytotoxic compounds delivered using the nanotransporters of the invention include, among others, mononucleosis.

In therapeutic applications, compositions are administered to a patient already affected by the particular disease, in an amount sufficient to cure or at least partially arrest the condition and its complications. An amount adequate to accomplish this is defined as a "therapeutically effective dose" or "efficacious dose". Amounts effective for this use will depend upon the severity of the condition, the general state of the patient, and the route of administration. Suspensions of infectious adenovirus particles may be delivered by various routes, including intravenous, intraperitoneal, intramuscular, subdermal, and topical. An adenovirus suspension containing about 10³ to 10¹² or more virion particles per ml may be administered by infusion or other suitable route. The nanotransporters of the invention can be used for the specific delivery of cytotoxic compounds such as clodronate, anthrax toxin, Pseudomonas exotoxin, typically modified so that these cytotoxic moieties do not bind to normal cells, and other toxins or cytotoxic agents including art-recognized chemotherapeutic agents such as adrenocorticoids, alkylating agents, antiandrogens, antiestrogens, androgens, estrogens, antimetabolites such as cytosine arabinoside, purine analogs, pyrimidine analogs, and methotrexate, busulfan, carboplatin, chlorambucil, cisplatin and other platinum compounds, tamoxiphen, taxol, cyclophosphamide, plant alkaloids, prednisone, hydroxyurea, teniposide, and bleomycin, nitrogen mustards, nitrosureas, vincristine, vinblastine, MEK kinase inhibitors, MAP kinase pathway inhibitors, PI-3-kinase inhibitors, mitochondrial perturbants, NFKB pathway inhibitors, proteosome inhibitors, pro- apoptotic agents, glucocorticoids, such as prednisolone, flumethasone, dexamethasone, and betamethasone, indomethacin, diclofenac, proteins such as pokeweed, saporin, momordin, and gelonin, non-steroidal anti-inflammatory drugs (NSAIDs), protein synthesis inhibitors, didemnin B, verrucarin A, geldanamycin, and the like.

Adenoviral therapy using the adenoviruses of the instant invention may be combined with concurrent or sequential treatment with other antineoplastic protocols, such as conventional chemotherapy, X-ray therapy to treat a particular cancer. Suitable therapeutic agents for forming immunoconjugates useful for the present invention include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, actinomycin D, 1-dehydro- testosterone, glucocorticoids, procaine, tetracaine, lidocaine, propranolol, and puromycin, antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTlC), procarbazine, mitomycin C, cisplatin and other platinum derivatives, such as carboplatin), antibiotics (such as dactinomycin (formerly actinomycin), bleomycin, daunorubicin (formerly daunomycin), doxorubicin, idarubicin, mithramycin, calicheamicin, mitomycin, mitoxantrone, plicamycin, anthramycin (AMC)), diphtheria toxin and related molecules (such as diphtheria A chain and active fragments thereof and hybrid molecules), ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Therapeutic agents, which may be administered in combination with an antibody as described elsewhere herein, may also be candidates for therapeutic moieties useful for conjugation to an antibody used in the present invention. Moreover, if the cytotoxic compound is a polypeptide, this include, without limitation, an enzymatically active toxin, or active fragment thereof, such as abrin, ricin A, pseudomonas exotoxin, or diphtheria toxin; a protein such as tumor necrosis factor or interferon-gamma or, biological response modifiers such as, for example, lymphokines, interleukin-1 (IL-1), interleukin-2 (IL-2), interleukin-6 (IL-6), granulocyte macrophage colony stimulating factor (GM-CSF), granulocyte colony stimulating factor (G-CSF), or other growth factors and apotopic inducing protein isolated from mitochondria.

Adenoviral therapy using the adenoviruses of the instant invention as adenoviral vectors may also be combined with other genes known to be useful in viral based therapy. See U. S. Patent No. 5,648,478. In such cases, the chimeric adenovirus further comprises a heterologous gene that encodes a therapeutic protein, incorporated within the viral genome, such that the heterologous gene is expressed within an infected cell. A therapeutic protein, as used herein, refers to a protein that would be expected to provide some therapeutic benefit when expressed in a given cell. In one embodiment, the heterologous gene is a prodrug activator gene, such as cytosine deaminase (CD) (See, U.S. Patent Nos. 5,631 ,236; 5,358,866; and 5,677,178). In other embodiments, the heterologous gene is a known inducer of cell-death, e.g apoptin or adenoviral death protein (ADP), or a fusion protein, e.g. fusogenic membrane glycoprotein (Danen-Van Oorschot et al. (1997) Proc. Nat. Acad. Sci. 94:5843-5847; Tollefson et al.(1996) J. Virol. 70:2296- 2306; Fu et al. (2003) MoI. Therapy 7: 48-754, 2003; Ahmed et al. (2003) Gene Therapy 10:1663-1671 ; Galanis et al. (2001) Human Gene Therapy 12(7): 811-821). Further examples of heterologous genes, or fragments thereof, include those that encode immunomodulatory proteins, such as cytokines or chemokines. Examples include interleukin 2, U.S. Patent Nos. 4,738,927 or 5,641, 665; interleukin 7, U. S. Patent Nos. 4,965,195 or 5,328,988; and interleukin 12, U. S. Patent No. 5,457,038; tumour necrosis factor alpha, L). S. Patent Nos. 4,677,063 or 5,773,582; interferon gamma, U.S. Patent Nos. 4,727,138 or 4,762,791 ; or GM CSF, U.S. Patent Nos. 5,393,870 or 5,391 ,485, Mackensen et al. (1997) Cytokine Growth Factor Rev. 8:119-128). Additional immunomodulatory proteins further include macrophage inflammatory proteins, including MIP- 3. Monocyte chemotatic protein (MCP-3 alpha) may also be used; a preferred embodiment of a heterologous gene is a chimeric gene consisting of a gene that encodes a protein that traverses cell membranes, for example, VP22 or TAT, fused to a gene that encodes a protein that is preferably toxic to cancer but not normal cells.

The chimeric adenoviruses of the invention can also be used as vectors to deliver genes encoding therapeutically useful RNA molecules, i.e. siRNA (Dorsett and Tuschl (2004) Nature Rev Drug Disc 3:318-329). In some cases, genes can be incorporated into a chimeric adenovirus of the invention to further enhance the ability of the oncolytic virus to erradicate the cell of the monocyte/macrophage lineage, although not having any direct impact on the cell itself. These include genes encoding siRNAs capable of inhibit the activity of factors that compromise MHC class I presentation, block complement, inhibit IFNs and IFN-induced mechanisms, chemokines and cytokines, NK cell based killing, down regulate the immune response (e.g. IL-10, TGF-Beta) and metalloproteases which can breakdown the extracelluar matrix and enhance spread of the virus within the tumour.

In an alternative embodiment, described is the use of the nanotransporters for the treatment of diseases associated with an increased production of IL-11 by cells of the monocyte-macropahge lineage and, in particular, by CD14+ monocytes. A non-exclusive list of acute and chronic IL-11-mediated diseases includes, but is not limited to, acute pancreatitis; ALS; Alzheimer's disease; cachexia/anorexia, including AIDS-induced cachexia; asthma and other pulmonary diseases; atherosclerosis; autoimmune vasculitis; chronic fatigue syndrome; Clostridium associated illnesses, including Clostridium-associated diarrhea; coronary conditions and indications, including congestive heart failure, coronary restenosis, myocardial infarction, myocardial dysfunction (e.g., related to sepsis), and coronary artery bypass graft; cancer, such as multiple myeloma and myelogenous (e.g., AML and CML) and other leukemias, as well as tumour metastasis; diabetes (e.g., insulin diabetes); endometriosis; fever; fibromyalgia; glomerulonephritis; graft versus host disease/transplant rejection; hemohorragic shock; hyperalgesia; inflammatory bowel disease; inflammatory conditions of a joint, including osteoarthritis, psoriatic arthritis and rheumatoid arthritis (RA); inflammatory eye disease, as may be associated with, for example, corneal transplant; ischemia, including cerebral ischemia (e.g., brain injury as a result of trauma, epilepsy, hemorrhage or stroke, each of which may lead to neurodegeneration); Kawasaki's disease; learning impairment; lung diseases (e.g., ARDS); multiple sclerosis; myopathies (e.g., muscle protein metabolism, esp. in sepsis); neurotoxicity (e.g., as induced by HIV); osteoporosis; pain, including cancer-related pain; Parkinson's disease; periodontal disease; pre-term labor; psoriasis; reperfusion injury; septic shock; side effects from radiation therapy; temporal mandibular joint disease; sleep disturbance; uveitis; inflammatory conditions resulting from strain, sprain, cartilage damage, trauma, orthopedic surgery, infection or other disease processes.

In an alternative embodiment, described is the use of the nanotransporters for the treatment of diseases associated with an increased production of TNF-α by cells of the monocyte-macropahge lineage and, in particular, by CD14+ monocytes. A non-exclusive list of diseases associated with an increased TNF-α activity include cachexia/anorexia; cancer (e.g., leukemias); chronic fatigue syndrome; coronary conditions and indications, including congestive heart failure, coronary restenosis, myocardial infarction, myocardial dysfunction (e.g., related to sepsis), and coronary artery bypass graft; depression; diabetes, including juvenile onset Type 1, diabetes mellitus, and insulin resistance (e.g., as associated with obesity); endometriosis, endometritis, and related conditions; fibromyalgia or analgesia; graft versus host rejection; hyperalgesia; inflammatory bowel diseases, including Crohn's disease and Clostridium difficile-associated diarrhea; ischemia, including cerebral ischemia (brain injury as a result of trauma, epilepsy, hemorrhage or stroke, each of which may lead to neurodegeneration); lung diseases (e.g., adult respiratory distress syndrome, asthma, and pulmonary fibrosis); multiple sclerosis; neuroinflammatory diseases; ocular diseases and conditions, including corneal transplant, ocular degeneration and uveitis; pain, including cancer-related pain; pancreatitis; periodontal diseases; Pityriasis rubra pilaris (PRP); prostatitis (bacterial or non-bacterial) and related conditions; psoriasis and related conditions; pulmonary fibrosis; reperfusion injury; rheumatic diseases, including rheumatoid arthritis, osteoarthritis, juvenile (rheumatoid) arthritis, seronegative polyarthritis, ankylosing spondylitis, Reiter's syndrome and reactive arthritis, Still's disease, psoriatic arthritis, enteropathic arthritis, polymyositis, dermatomyositis, scleroderma, systemic sclerosis, vasculitis (e.g., Kawasaki's disease), cerebral vasculitis, Lyme disease, staphylococcal-induced ("septic") arthritis, Sjogren's syndrome, rheumatic fever, polychondritis and polymyalgia rheumatica and giant cell arteritis); septic shock; side effects from radiation therapy; systemic lupus erythematosus (SLE); temporal mandibular joint disease; thyroiditis; tissue transplantation or an inflammatory condition resulting from strain, sprain, cartilage damage, trauma, orthopedic surgery, infection (e.g., HIV, Clostridium difficile and related species) or other disease process.

It will be appreciated that diseases associated with increased production of IL-1 or TNF-α may be treated using cytotoxic compounds in order to decrease the number of viable monocytes or, alternatively, it is possible to target specifically IL-1 or TNF-α by using compounds capable of specifically inhibiting or reducing the synthesis of this cytokines. Thus, the nanotransporters of the invention may comprise IL-1 and TNF-α specific inhibitors such as IL-1 or TNF-α specific antisense oligonucleotides, ribozymes, siRNAs, aptamers and the like or polynucleotides encoding said inhibitors. It will be understood that if the nanotransporters comprises a polynucleotide encoding an inhibitory compound, it will be ideally under the control of a monocyte-specific promoter such as the CD11b promoter.

The medical uses described herein contemplate the use of any of the nanotransporters mentioned above, including virus, a viral-like particle, a nanoparticle or a protein cage carrying a adenovirus subgroup F short fiber protein such as the short fibre protein of Ad40 or the short fiber protein of Ad41. In a still more preferred embodiment, the adenoviral particle is an Ad5 and the short fiber protein is the Ad40 short fiber protein.

The present invention refers to a nanotransporter comprising a product of interest and at least part of the knob domain of the short fiber protein of adenovirus Ad40 or Ad41 for inducing an immune response by delivering an antigen or part thereofthe treatment of a disease associated with cells of the monocyte-macrophage lineage, wherein the nanotransporter is selected from the group of a virus or a viral-like particle and wherein said virus or viral-like particle is not an adenovirus subgroup F of the same type as the short fiber protein.

The present invention also refers to a nanotransporter comprising a product of interest and at least part of the knob domain of the short fiber protein of adenovirus Ad40 or Ad41 for reducing an increased immune response by delivering cytotoxic compounds, wherein the nanotransporter is selected from the group of a virus or a viral-like particle and wherein said virus or viral-like particle is not an adenovirus subgroup F of the same type as the short fiber protein.

The present invention also refers to a nanotransporter comprising a product of interest and at least part of the knob domain of the short fiber protein of adenovirus Ad40 or Ad41 for reducing inflammation by delivering an anti-inflammatory component, wherein the nanotransporter is selected from the group of a virus or a viral-like particle and wherein said virus or viral-like particle is not an adenovirus subgroup F of the same type as the short fiber protein.

The invention is described by way of the following examples which are to be construed as merely illustrative and not limitative of the scope of the invention.

### EXAMPLES

### MATERIALS AND METHODS

### Recombinant Ad5/40

Recombinant Ad5/40 were obtained by replacing the whole Ad5 fiber protein by the complete Ad40 short fiber.

### Virus amplification

HEK-293 cells (Q-BIOgene, Montreal, Canada) were grown in DMEM medium (#E15-810, PAA laboratories, Linz, Austria) and 10% of fetal bovine serum (FBS) (PAA laboratories, Linz, Austria).

Virus stocks were sequentially amplified from one 10-cm plate (10⁷ cells) were amplified in twenty 15-cm plates (5x10⁸ cells) for 36h (Ad5) to 60 hours (Ad5/40) or until cytopathic effect was observed. Total amplification steps lasted for 7-10 days for Ad5 vectors, and for 30-45 days for Ad5/40 vectors.

In all cases, at the final amplification step, the cell pellet was concentrated to a final volume of 40-45 ml, and three freeze/thaw rounds were performed to liberate virus particles. Supernatant was centrifuged in two consecutive CsCl gradients [(a) step gradient of 1.40 g/cm³- 1.25 g/cm³; and (b) an isopycnic gradient of 1.35 g/cm³] to purify viral particles and desalted using a Sephadex PD-10 desalting column (Amersham Biosciences, Uppsala, Sweden) to remove CsCl.

Final purified viral stocks were titered by determining their concentration (particles/ml) by optical density at 260 nm (1 OD260 unit=1.1x10¹² particles/ml), and their infectivity (infectious units/ml) was measured by endpoint dilution assay. Briefly, end-point dilution assays were performed in triplicate by infecting 293 cells with serially diluted vectors, and then counting the number of transgene (GFP or βgal) expressing cells after 24 h (Alba et al). The particle to infectious unit ratio of adenovirus used in the experiments had an average of 15:1 for Ad5 and 100:1 to 500:1 for Ad5/40.

All the vectors produced were analyzed to test absence of bacteria, funghi, yeast and mycoplasm contamination.

### Cell lines and culture

The monocyte-macrophage mouse cell line RAW 264.7 was grown on DMEM (#41965, Gibco Invitrogen Ltd, Paisley, UK) + 10 % FBS (#10106-169, Gibco Invitrogen Ltd, Paisley, UK) + 2 % Penicillin-Streptomicin (Pen/Strep) solution (#15070-063, Gibco Invitrogen Ltd, Paisley, UK). The human monocyte U-937 and THP-1 cell lines were grown on RPMI 1640 (#21875-034, Gibco Invitrogen Ltd, Paisley, UK) + 10 % FBS + 2 % Pen/Strep.

### Transduction of cell lines

Human monocyte cell lines THP-1 and U-937 were grown in absence of any differentiation factor, such as LPS, PMA, etc, to avoid attachment of cells to substrate and differentiation of cells to a macrophage phenotype. For transduction analysis of THP-1 and U-937 cells expanded in culture were collected and viable cells were counted using the trypan blue exclusion method. Cells were then seeded in infection medium: DMEM + 2% FBS + 1% NEAA (#11140, Gibco Invitrogen Ltd, Paisley, UK) + 2% Pen/Strep. at a density of 750.000 cells/well in 6 wells plates. Immediately after seeding, 100 µl of infection medium or 100 µl of infection medium containing 250 physical particles/cell of the Ad5/40 vector or 100 µl of infection medium containing 250 physical particles/cell of the Ad5 vector were added to the culture.

For the transduction of the mouse adherent RAW 264.7 cell line, cells expanded in culture were detached from the substrate using a cell scrapper and viable cells were counted as described above. Cells were then seeded in grown medium at a density of 750.000 cells/well in 6 wells plates and allowed to attach for 24 h. After cell attachment, grown medium was replaced by infection medium and 100 µl of infection medium or 100 µl of infection medium containing 250 physical particles/cell of the Ad5/40 vector or 100 µl of infection medium containing 250 physical particles/cell of the Ad5 vector were added to the culture.

Plates were then gently swirled to uniformly distribute the adenoviral vector in the culture. All plates were incubated at 37 °C in a 5 % CO₂ atmosphere and GFP expression was monitored every 24 h. in a fluorescent inverted microscope.

### Flow cytometry analysis of cell lines

Two days after transduction, supernatant cells of THP-1 and U-937 were collected and placed in 1.5 ml centrifuge tubes. Cells were then centrifuged at 1000 X g for 5 minutes at room temperature. After centrifugation cells were washed with 1 ml of 1 X PBS and tubes were centrifuged again at 1000 X g for 5 minutes at room temperature. The supernatant fraction was gently removed and the cell pellet was suspended in 300 µl of 1 X PBS + 2 % formaldehyde.

Cells of the adherent cell line RAW 264.7 were collected by cell scrapping two days after transduction and processed as described above for THP-1 and U-937.

Cell lines were analyzed by flow cytometry in a BD FACSCANTO II flow cytometer (RAW 264.7 and U-937) and BD FACSCalibur flow cytometer (THP-1)

### Isolation and culture of human peripheral blood mononuclear cells (PBMC)

Forty five ml of blood obtained from buffy coats of healthy human donors was centrifuged at 300 X g for 15 minutes at room temperature. The plasma fraction was retired and total volume was reestablished by addition of 1 X PBS + 0.5 % FBS. This wash step was repeated once more. After finish the wash steps, blood was diluted 1:1 with 1 X PBS + 0.5 % FBS. Thirty mililiters of diluted blood were placed over a 15 ml of Lymphoprep (#1114544 AXIS-SHIELD PoC AS, Oslo, Norway) in a 50 ml centrifuge tube. Blood was then centrifuged at 800 X g for 20 minutes at room temperature. After centrifugation, tubes were fixed in a metallic support and the PBMC layer was carefully removed using an extra-long sterile 1.250 ml filter tip subjected to a 1 ml pipette. The recovered PBMC were placed in a 50 ml centrifuge tube and diluted with 1 X PBS to a final volume of 45 ml. Tubes were then inverted to wash the cells. Diluted PBMC were then centrifuged a 400 X g for 5 minutes at room temperature and supernatant fraction was discarded without disturbing the cell pellet. This wash step was repeated once more, but this time a volume of 2-3 ml of the supernatant fraction was left in the tube. The PBMC were suspended in this volume and 20 µl of the cellular suspension were diluted 1:10 in 1 X PBS. Ten microliters of this dilution were further diluted 1:1 with trypan blue dye (#T8154, SIGMA, Munich, Germany). Viable cells were counted in a Neubauer haemocytometer under an inverted microscope using a phase contrast filter (x 100). Viable cells of four different squares of 1 mm square area were counted and arithmetic mean was calculated. This mean was used to calculate the cell concentration (cells/ml) in the PBMC solution and then cells were diluted in infection medium: DMEM + 2% FBS + 1% NEAA + 2% Pen/Strep to obtain a final concentration of 1.034.483 cells/ml. After dilution, cells were countered again as stated before to ascertain that cell concentration was between a range from 1.006.897 to 1.062.069 cells/ml. Seven hundred and fifty microliters of the PBMC suspension were seeded per well (750.000 cells/well) in 6 wells plates.

### Antibodies

Antibodies against human monocyte cells: anti-CD14-APC (#345787, BD Biosciences, Erembodegem, Belgium) for the determination of CD14+ cells together with the violet-fluorescent reactive dye (#L34955, Invitrogen Ltd, Paisley, UK) for the discrimination of viable and non-viable cells were used for the citotoxicity analysis.

For the transduction specificity analysis a mixed solution of antibodies against human cells containing: anti-CD3-PerCP (#345766, BD Biosciences, Erembodegem, Belgium), anti-CD16-PE (Leu-11c) (#332779, BD Biosciences, Erembodegem, Belgium), anti-CD19-APC-Cy7 (#348814, BD Biosciences, Erembodegem, Belgium), anti-CD56-PE (#345810, BD Biosciences, Erembodegem, Belgium) and anti-CD14-APC (#345787, BD Biosciences, Erembodegem, Belgium) were used.

### Cytotoxicity analysis

Seven hundred and fifty microliters of PBMC were seeded per well (750.000 cells/well) in 6 wells plates. Immediately after seeding, 100 µl of infection medium or 100 µl of infection medium containing different amounts of physical particles of the Ad5/40 vector or 100 µl of infection medium containing different amounts of physical particles of the Ad5 vector were added to the culture. Plates were gently swirled to uniformly distribute the adenoviral vector in the culture. All plates were incubated at 37 °C in a 5 % CO₂ atmosphere and GFP expression was monitored every 24 h. in a fluorescence inverted microscope.

### Transduction specificity analysis

Seven hundred and fifty microliters of PBMC were seeded per well (750.000 cells/well) in 6 wells plates. Immediately after seeding, 100 µl of infection medium or 100 µl of infection medium containing 250 physical particles/cell of the Ad5/40 vector or 100 µl of infection medium containing 250 physical particles/cell of the Ad5 vector added to the PBMC culture. Plates were gently swirled to uniformly distribute the adenoviral vector in the culture. All plates were incubated at 37 °C in a 5 % CO₂ atmosphere and GFP expression was monitored every 24 h. in a fluorescence inverted microscope.

### Flow citometry analysis of PBMC

Two days after transduction, supernatant PBMC were collected and placed in 1.5 ml centrifuge tubes. Cells were then centrifuged at 1000 X g for 5 minutes at room temperature. After centrifugation, supernatant was gently removed and the cell pellet was suspended in 100 µl of a 1 X PBS solution containing the corresponding antibodies. Tubes containing the PBMC suspended in this solution were then incubated for 15-20 minutes in the dark at room temperature. After incubation, 1 ml of 1 X PBS was added to each tube and tubes were centrifuged at 1000 X g for 5 minutes at room temperature. After centrifugation, supernatant was gently removed and the cell pellet was suspended in 300 µl of 1 X PBS + 2 % formaldehyde. Cell populations were then analyzed in a BD FACSCANTO II flow cytometer.

### EXAMPLE 1

### Ad5/40 is capable of transfecting intestinal mucose mouse macrophages

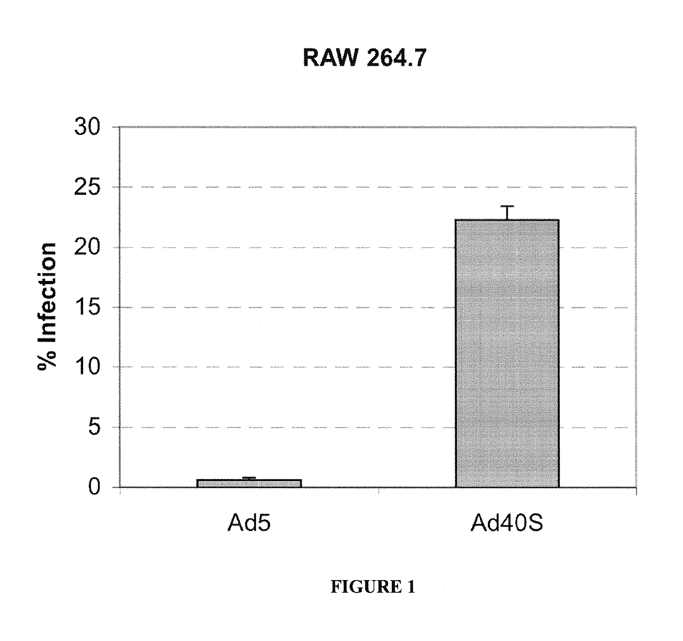

Recombinant Ad5/40-GFP were administered orally and intrarectally to mice. Green fluorescence was observed at the submucosal level in intestinal tissue sections. Since the presence of resident macrophages in this area is common even in healthy animals, we decided to assess whether Ad5/40 could efficiently transfect mouse macrophage cell lines. RAW 264.7 cells were cultured and infected with Ad5/40-GFP at 250 PP/cell (Figure 1). Interestingly, the efficiency of infection was clearly greater than that of the Ad5 at the same conditions. Therefore, the results confirmed that Ad5/40 could efficiently transfect mouse macrophages.

### EXAMPLE 2

### Ad5/40 is capable of transfecting human monocyte-derived macrophages

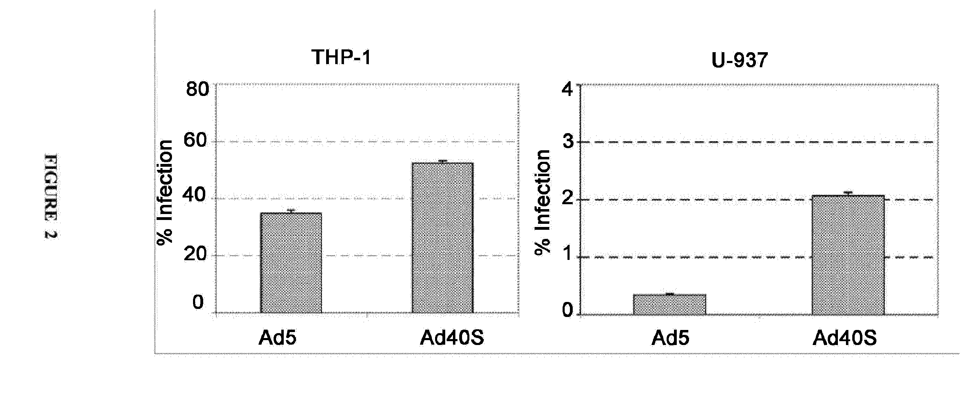

Next, the ability of Ad5/40 to efficiently transfect human monocyte derived macrophages was tested. For this purpose THP1 (good infectivity using Ad5) and U-937 cells (very low infectivity using Ad5) were cultured in the absence of any differentiation factor that might induce maduration of monocytes to macrophages (such as LPS, PMA, etc). At this stage, the culture medium was changed to infection medium and 250 physical particles/cell of Ad5/40-GFP or 250 physical particles/cell of Ad5-GFP were added. Flow cytometry analysis of the supernatant fraction of the different cultures revealed higher percentage of transduced cells and higher GFP expression in the cultures infected by the Ad5/40 vector after 48h, thus confirming the superior efficiency of this vector in the transfection of human monocyte cell lines (Figure 2).

### EXAMPLE 3

### Ad5/40 is capable of transfecting peripheral blood monocytes

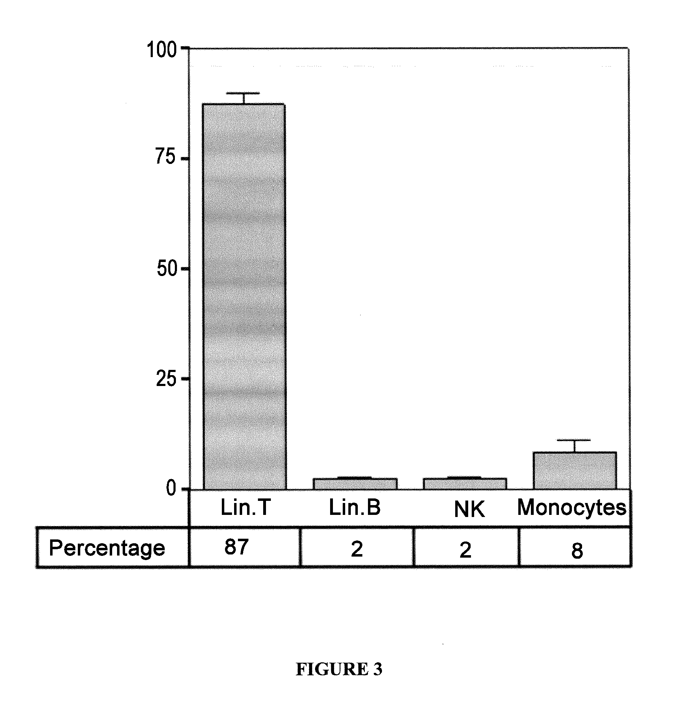

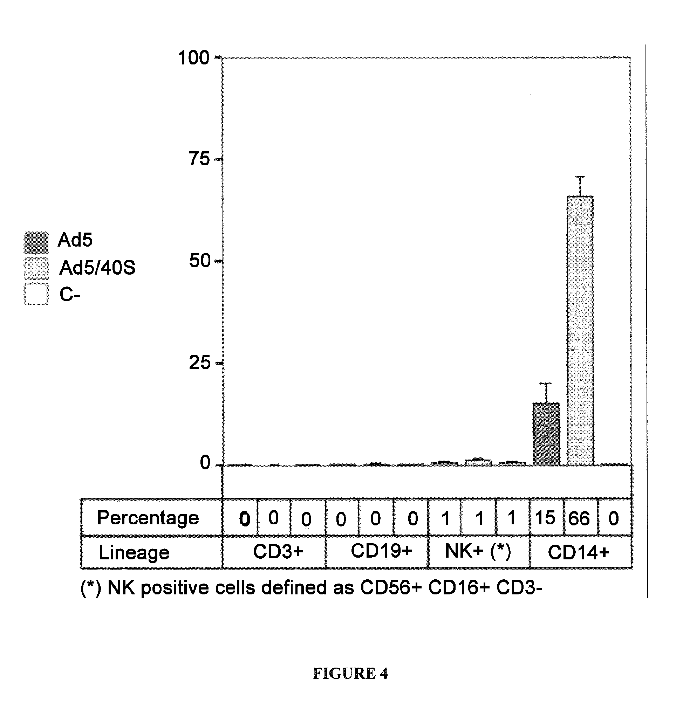

Next, it was tested whether Ad5/40 was able to transfect peripheral blood monocytes. For this purpose mononuclear cells (i.e lymphocytes, monocytes and NK cells) were obtained from buffy coats from healthy human donor's blood. Interestingly, the Ad5/40 was able to selectively infect peripheral blood monocytes, despite the fact that they represent on average only 8% of total mononuclear blood cells (Figure 3). In fact, at a dose of 250 physical particles per cell, infection is not only more efficient in the case of Ad5/40 compared to Ad5 (66% vs. 15%) (Figure 4), but was extremely selective as more than 99.9% of the Ad5/40 infected exclusively CD14+ cells (monocytes).

### EXAMPLE 4

### Transduction of monocytes by Ad5/40 depends exclusively on CD14 and not on CD16

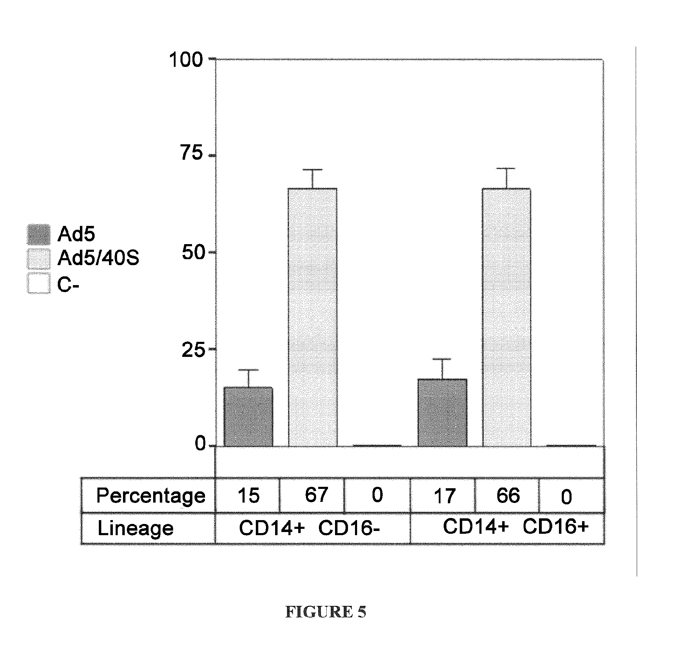

In order to better characterize the differences in the infectivity between Ad5 and Ad5/40, non-saturating viral load conditions were used (250 viral particles per blood cell). However, in these conditions it is possible to determine whether any of the subpopulations of monocytes, such as pro-inflammatory CD14+/CD16+ versus classical CD14+/CD16-, is selectively infected. As shown in Figure 5, the infectivity of Ad5/40 is not associated to the expression of CD16: Ad5 (15 ± 2% versus 17% ± 3); Ad5/40 (67% ± 3 versus 66% ± 2). This is an important point because both CD14+/CD16 populations have important therapeutic applications, and therefore, the ability of Ad5/40 to efficiently infect the two peripheral blood monocyte subsets implies a great potential of Ad5/40 as a tool for cell specific gene transfer.

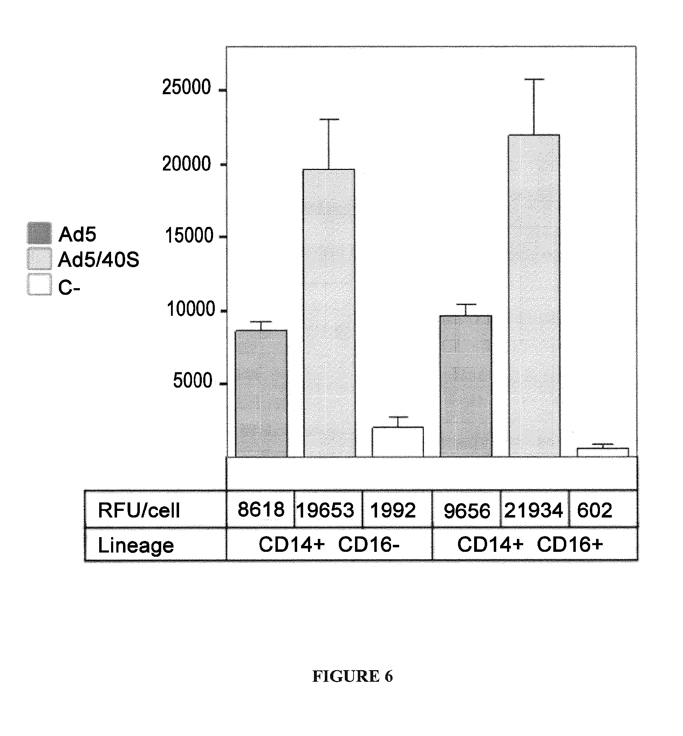

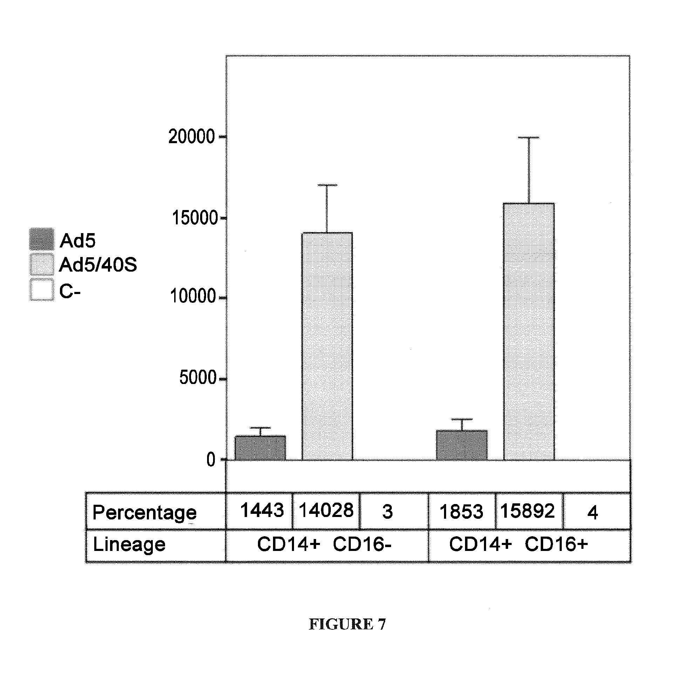

A more detailed analysis of the Ad5/40 infectivity in cells CD14+/CD16- and CD14+/CD16+ shows that the level of transgene expression per infected cell is significantly higher (around 200%) for Ad5/40 than for the Ad5 (Figure 6), which together with the percentages of infected cells means that the amount of protein expressed is about 10 times higher for Ad5/40 that for the Ad5 (Figure 7). In both cases, GFP expression is slightly higher in (CD14+/CD16+) monocytes, although there are not significant differences compared to classical (CD14 +/ CD16-) monocytes.

### EXAMPLE 5

### Transduction of CD14+ cells by Ad5/40 does not result in significant loss of cell viability

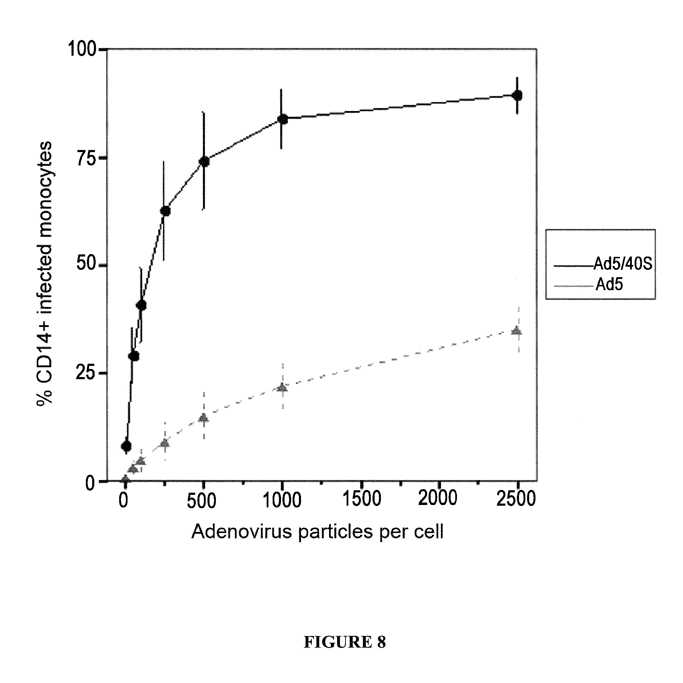

To test the relative cytotoxicity of Ad5/40 vs Ad5 a dose-response experiment was performed. Mononuclear cells from human peripheral blood were incubated with increasing viral doses. As shown in Figure 8, in all conditions tested, Ad5/40 is clearly more efficient at infecting CD14+ cells than Ad5. Thus Ad5/40 infectivity plateaus when close to 90% of total monocytes are infected, while for Ad5, saturation is not observed even at very high doses (2500 physical particles per cell). Analysis of the two dose:response curves shows that Ad5/40 is about 25-50 times more efficient for gene transfer to CD14+ cells than Ad5, since to infect 33% of monocytes a dose of 2500 Ad5 particles per cell is necessary, whereas doses of 50-100 Ad5/40 particles per cell are enough to equal this infection level. The dose of Ad5/40 used in most experiments (250 particles per cell) is just below the stage of saturation or plateau.

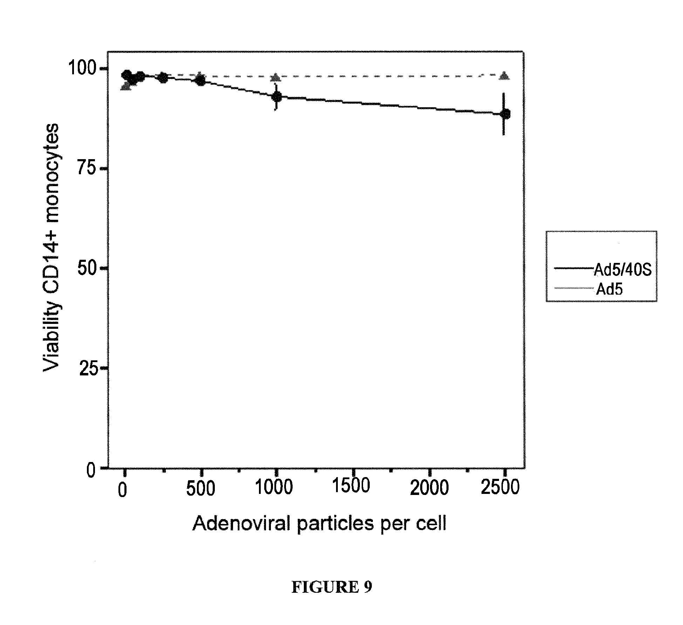

The low efficiency of Ad5 to infect CD14+ cells could explain why at the highest conditions used (2500 particles per cell) viability of CD14+ cells is hardly affected. On the contrary, Ad5/40 at high doses (1000 and 2500 viral particles per cell) is associated with a decline in the viability of CD14+ cells, probably due to cytotoxicity caused by the high viral load (Figure 9).

## Claims

1. An in vitro method for delivering a compound of interest to CD14+ cells derived from monoblast which comprises contacting said cells with a nanotransporter carrying said compound of interest, wherein the nanotransporter contains at least the knob domain of the short fiber protein of adenovirus Ad40 or Ad41, wherein the nanotransporter is selected from the group of a virus or a viral-like particle and wherein said virus or viral-like particle is not an adenovirus subgroup F of the same type as the short fiber protein.

2. A method as defined in claim 1, wherein the nanotransporter is an adenoviral particle, preferably an Ad5.

3. A method as defined in claim 1 or 2, wherein the the compound of interest is delivered to a cell of the monocyte-macrophage lineage selected from a primary mononuclear blood cell or a monocyte-derived dendritic cell.

4. A method as defined in any of the preceding claims, wherein the product of interest is selected from the group comprising therapeutic agents, diagnostic agents, prophylactic agents, and/or nutraceutical agents.

5. A method as defined in claim 4 wherein the product of interest to be delivered is an small molecule, a nucleic acid, such as cDNAs, siRNA, RNAi, and microRNA agents or natural or unnatural shRNA, ribozymes, DNA plasmids, aptamers, antisense oligonucleotides, randomized oligonucleotides; a protein, a peptide, a lipid, a carbohydrate, a hormone, a metal, a radioactive element and compound, a drug, a vaccine, an immunological agent and/or combinations thereof.

6. Nanotransporter comprising a product of interest and at least part of the knob domain of the short fiber protein of adenovirus Ad40 or Ad41 for inducing an immune response by delivering an antigen, wherein the nanotransporter is selected from the group of a virus or a viral-like particle and wherein said virus or viral-like particle is not an adenovirus subgroup F of the same type as the short fiber protein.

7. Nanotransporter comprising a product of interest and at least part of the knob domain of the short fiber protein of adenovirus Ad40 or Ad41 for reducing an increased immune response by delivering cytotoxic compounds, wherein the nanotransporter is selected from the group of a virus or a viral-like particle and wherein said virus or viral-like particle is not an adenovirus subgroup F of the same type as the short fiber protein.

8. Nanotransporter comprising a product of interest and at least part of the knob domain of the short fiber protein of adenovirus Ad40 or Ad41 for reducing inflammation by delivering an anti-inflammatory component, wherein the nanotransporter is selected from the group of a virus or a viral-like particle and wherein said virus or viral-like particle is not an adenovirus subgroup F of the same type as the short fiber protein.

## Patentansprüche

1. In-vitro-Verfahren zum Liefern einer Verbindung von Interesse zu CD14+ Zellen, welche von Monoblast abgeleitet ist, welches Kontaktieren der Zellen mit einem Nanotransporter, welcher die Verbindung von Interesse trägt, umfasst, wobei der Nanotransporter wenigstens die Knopfdomäne (knob domain) von dem Kurzfaserprotein vom Adenovirus AD40 oder AD41 beinhaltet, wobei der Nanotransporter ausgewählt ist aus der Gruppe von einem Virus oder einem virusähnlichen Teilchen und wobei der Virus oder das virusähnliche Teilchen kein Adenovirus Untergruppe F von demselben Typ wie das Kurzfaserprotein ist.

2. Verfahren gemäß Anspruch 1, wobei der Nanotransporter ein adenovirales Teilchen ist, vorzugsweise ein Ad5.

3. Verfahren gemäß Anspruch 1 oder 2, wobei die Verbindung von Interesse an eine Zelle von der monozyt-makrophagen Abstammungslinie geliefert wird, welche von einer primären mononuklearen Blutzelle oder einer monozyt-abgeleiteten dendritischen Zelle ausgewählt ist.

4. Verfahren gemäß einem der vorangehenden Ansprüche, wobei das Produkt von Interesse ausgewählt ist aus der Gruppe umfassend therapeutische Mittel, diagnostische Mittel, prophylaktische Mittel, und/oder nutrazeutische Mittel.

5. Verfahren gemäß Anspruch 4, wobei das Produkt von Interesse, welches geliefert werden soll, ein kleines Molekül ist, eine Nukleinsäure, so wie cDNAs, siRNA, RNAi und mikroRNA Mittel oder natürliche oder unnatürliche shRNA, Ribozyme, DNA Plasmide, Aptamere, Antisense-Oligonukleotide, randomisierte Oligonukleotide; ein Protein, ein Peptid, ein Lipid, ein Kohlenhydrat, ein Hormon, ein Metall, ein radioaktives Element und Verbindung, ein Arzneimittel, ein Impfstoff, ein immunologisches Mittel und/oder Kombinationen davon.

6. Nanotransporter, umfassend ein Produkt von Interesse und wenigstens einen Teil von der Knopfdomäne von dem Kurzfaserprotein von Adenovirus AD40 oder AD41 zum Induzieren einer Immunantwort durch Liefern eines Antigens, wobei der Nanotransporter ausgewählt ist aus der Gruppe von einem Virus oder einem virusähnlichen Teilchen und wobei das Virus oder virusähnliche Teilchen nicht ein Adenovirus Untergruppe F von demselben Typ wie das Kurzfaserprotein ist.

7. Nanotransporter, umfassend ein Produkt von Interesse und wenigstens einen Teil von der Knopfdomäne von dem Kurzfaserprotein von Adenovirus AD40 oder AD41 zum Reduzieren einer erhöhten Immunantwort durch Liefern zytotoxischer Verbindungen, wobei der Nanotransporter ausgewählt ist aus der Gruppe von einem Virus oder einem virusähnlichen Teilchen und wobei das Virus oder virusähnliche Teilchen nicht ein Adenovirus Untergruppe F von demselben Typ wie das Kurzfaserprotein ist.

8. Nanotransporter, umfassend ein Produkt von Interesse und wenigstens einen Teil von der Knopfdomäne von dem Kurzfaserprotein von Adenovirus AD40 oder AD41 zum Reduzieren von Entzündung durch Liefern einer entzündungshemmenden Komponente, wobei der Nanotransporter ausgewählt ist aus der Gruppe von einem Virus oder einem virusähnlichen Teilchen und wobei das Virus oder virusähnliche Teilchen kein Adenovirus Untergruppe F von demselben Typ wie das Kurzfaserprotein ist.

## Revendications

1. Procédé in vitro pour délivrer un composé d'intérêt à des cellules CD14+ dérivées de monoblaste, qui comprend la mise en contact desdites cellules avec un nanotransporteur transportant ledit composé d'intérêt, dans lequel le nanotransporteur contient au moins le domaine de tête de la protéine à fibres courtes de l'adénovirus Ad40 ou Ad41, dans lequel le nanotransporteur est sélectionné dans le groupe d'un virus ou d'une particule de type viral et dans lequel ledit virus ou ladite particule de type viral n'est pas un sous-groupe F d'adénovirus du même type que la protéine à fibres courtes.

2. Procédé tel que défini dans la revendication 1, dans lequel le nanotransporteur est une particule adénovirale, de préférence une Ad5.

3. Procédé tel que défini dans la revendication 1 ou 2, dans lequel le composé d'intérêt est délivré à une cellule de lignage monocyte-macrophage sélectionnée dans une cellule sanguine mononucléaire primaire ou une cellule dendritique dérivée de monocyte.

4. Procédé tel que défini dans l'une quelconque des revendications précédentes, dans lequel le produit d'intérêt est sélectionné dans le groupe comprenant des agents thérapeutiques, des agents de diagnostic, des agents prophylactiques, et/ou des agents nutraceutiques.

5. Procédé tel que défini dans la revendication 4 dans lequel le produit d'intérêt devant être délivré est une petite molécule, un acide nucléique, tels que les ADNc, l'ARNsi, l'ARNi, et les agents micro-ARN ou l'ARNsh naturel ou non-naturel, les ribozymes, les plasmides d'ADN, les aptamères, les oligonucléotides antisens, les oligonucléotides aléatoires ; une protéine, un peptide, un lipide, un hydrate de carbone, une hormone, un métal, un élément radioactif et un composé, un médicament, un vaccin, an agent immunologique et/ou des combinaisons de ceux-ci.

6. Nanotransporteur comprenant un produit d'intérêt et au moins une partie du domaine de tête de la protéine à fibres courtes de l'adénovirus Ad40 ou Ad41 pour induire une réponse immunitaire par la délivrance d'un antigène, dans lequel le nanotransporteur est sélectionné dans le groupe d'un virus ou d'une particule de type viral et dans lequel ledit virus ou ladite particule de type viral n'est pas un sous-groupe F d'adénovirus du même type que la protéine à fibres courtes.

7. Nanotransporteur comprenant un produit d'intérêt et au moins une partie du domaine de tête de la protéine à fibres courtes de l'adénovirus Ad40 ou Ad41 pour réduire une réponse immunitaire accrue par la délivrance de composés cytotoxiques, dans lequel le nanotransporteur est sélectionné dans le groupe d'un virus ou d'une particule de type viral et dans lequel ledit virus ou ladite particule de type viral n'est pas un sous-groupe F d'adénovirus du même type que la protéine à fibres courtes.

8. Nanotransporteur comprenant un produit d'intérêt et au moins une partie du domaine de tête de la protéine à fibres courtes de l'adénovirus Ad40 ou Ad41 pour réduire une inflammation par la délivrance d'un composant anti-inflammatoire, dans lequel le nanotransporteur est sélectionné dans le groupe d'un virus ou d'une particule de type viral et dans lequel ledit virus ou ladite particule de type viral n'est pas un sous-groupe F d'adénovirus du même type que la protéine à fibres courtes.
